# EUROPEAN PATENT APPLICATION

(11) **EP 4 029 875 A1**
(43) Date of publication of application: **20.07.2022**
(21) Application number: 20863270.3
(22) Date of filing: 11.09.2020
(51) Int. Cl.: C07K 14/415, C12N 15/82, C12N 9/88, C12N 15/60, A01H 5/00

(54) **HERBICIDE RESISTANT PLANT**

(30) Priority: 12.09.2019 CN 201910862733; 26.09.2019 CN 201910915259; 27.09.2019 CN 201910923052
(71) Applicant: INSTITUTE OF GENETICS AND DEVELOPMENTAL BIOLOGY, CHINESE ACADEMY OF SCIENCES, Chaoyang District Beijing 100101 (CN); Tianjin Genovo Biotechnology Co., Ltd., Tianjin 301700 (CN)
(72) Inventor: GAO, Caixia, Beijing 100101 (CN); CHEN, Yuhang, Beijing 100101 (CN); RAN, Yidong, Tianjin 301700 (CN); ZHANG, Rui, Beijing 100101 (CN); LI, Chao, Beijing 100101 (CN); CHEN, Sha, Beijing 100101 (CN); ZHANG, Kang, Tianjin 301700 (CN); XU, Hu, Tianjin 301700 (CN)
(74) Representative: Michalski Hüttermann & Partner Patentanwälte mbB
(86) International application number: PCT/CN2020/114874
(87) International publication number: WO 2021/047656

(57) **Abstract**

The present invention relates to the field of plant genetic engineering. In particular, the present invention relates to acetolactate synthase (ALS) mutants, protoporphyrinogen oxidase (PPO) mutants, acetyl-CoA carboxylase (ACCase) mutants and/or p-hydroxyphenylpyruvate dioxidase (HPPD) mutants capable of conferring herbicide resistance in plants, especially in wheat and/or rice plants, and methods for production herbicide-resistant plants, especially wheat and/or rice plants comprising said acetolactate synthase (ALS) mutants and/or protoporphyrinogen oxidase (PPO) mutants and/or acetyl-CoA carboxylase (ACCase) mutants and/or p-hydroxyphenylpyruvate dioxidase (HPPD) mutants.

## Description

### Technical field

The present invention relates to the field of plant genetic engineering. In particular, the present invention relates to acetolactate synthase (ALS) mutants, protoporphyrinogen oxidase (PPO) mutants, acetyl-CoA carboxylase (ACCase) mutants and/or p-hydroxyphenylpyruvate dioxidase (HPPD) mutants capable of conferring herbicide resistance in plants, especially in wheat and/or rice plants, and methods for production herbicide-resistant plants, especially wheat and/or rice plants comprising said acetolactate synthase (ALS) mutants and/or protoporphyrinogen oxidase (PPO) mutants and/or acetyl-CoA carboxylase (ACCase) mutants and/or p-hydroxyphenylpyruvate dioxidase (HPPD) mutants.

### Background

A major threat to the production of crops such as rice is weed competition, which leads to reduced grain yields and poor quality. Although farming can be used to eliminate weeds, the soil of cultivated land is susceptible to erosion by wind and water. Due to its ease of application and effectiveness, herbicide treatment is the preferred method of weed control. Herbicides can also be used for weed control in direct seeding planting systems that reduce arable land or are designed to leave high levels of residue on the soil surface to prevent erosion.

The development of herbicide resistance in plants provides important production and economic advantages; therefore, the use of herbicides to control weeds or undesirable plants in crops has almost become a common practice. However, the use of such herbicides can also cause the death or reduction of growth of the desired crop plants, which makes the timing and procedures of herbicide application very critical or in some cases simply not feasible. To solve this problem, one of the methods is to develop herbicide-resistant varieties. In this method, herbicides are applied to crops to control weeds without causing damage to the herbicide-resistant crops.

For farmers, it is of particular interest to use herbicides with greater potency, broad weed spectrum effectiveness, and rapid soil degradation. By allowing the use of herbicides to control weed growth without the risk of damaging crops, plants, plant tissues and seeds that are resistant to these compounds provide attractive solutions.

Broad-spectrum herbicides include those compounds that inhibit acetolactate synthase (ALS) activity, protoporphyrinogen oxidase (PPO) activity, acetyl-CoA carboxylase (ACCase) activity, or p-hydroxyphenylpyruvate dioxygenase (HPPD) activity in plants. However, wheat is susceptible to many ALS- or PPO-inhibiting herbicides which target monocots, while rice is susceptible to many ACCase- or HPPD-inhibiting herbicides which target monocots, making it nearly impossible to use these herbicides to control grass weeds.

Therefore, there remains a need in the art to develop crop plants, such as wheat and/or rice, that are resistant to ALS and/or PPO and/or ACCase and/or HPPD inhibiting herbicides.

### Description of the drawings

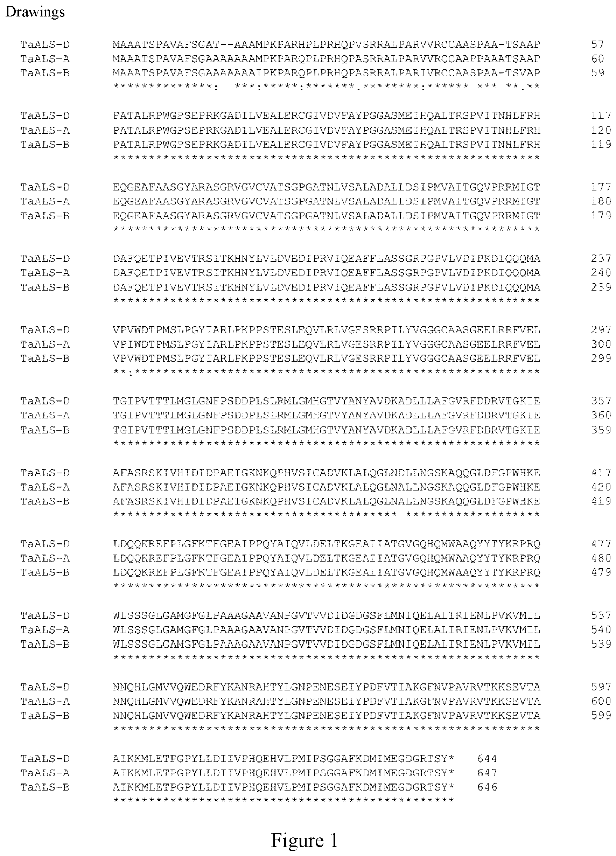

Figure 1 shows the amino acid sequence alignment of the ALS encoded by the wheat A, B and D genomes. This alignment can be used to determine, for example, the amino acid position numbering referring to the ALS amino acid sequence encoded by A genome.

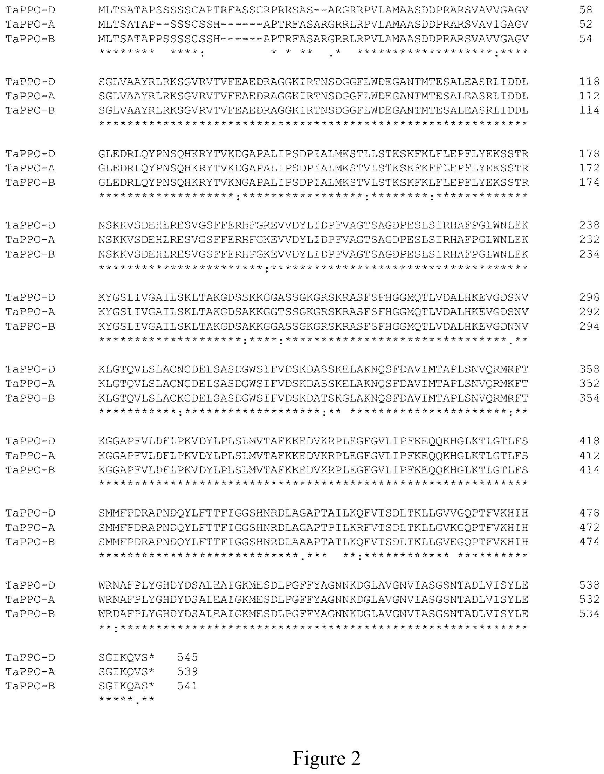

Figure 2 shows the amino acid sequence alignment of PPO encoded by the wheat A, B and D genomes. This alignment can be used to determine, for example, the amino acid position numbering referring to the PPO amino acid sequence encoded by the A genome.
Figure 3 shows rice resistant mutants surviving after spraying with haloxyfop-P-methyl. The surviving resistant mutants in the upper panel are S1866F heterozygous mutant, A1884P heterozygous mutant, and P2128L homozygous mutant; the surviving resistant mutants in the lower panel are P1927F homozygous and heterozygous mutants. The heart leaves of wild-type Zhonghua No. 11 (WT) in the upper and lower images were both dry and dead. The length of the coordinate ruler is 1cm.
Figure 4 shows the mutation types of the resistant mutants generated by targeting the four sites: OsACC-S1866, OsACC-A1884, OsACC-P2181 and OsACC-P1927.

### Detailed description of the invention

### 1. Definition

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely used in the corresponding field. For example, standard recombinant DNA and molecular cloning techniques used in the present invention are well known to those skilled in the art and are more fully described in the following document: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook"). In the meantime, in order to better understand the present invention, definitions and explanations of related terms are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

"Exogenous" in reference to a sequence means a sequence from a foreign species, or refers to a sequence in which significant changes in composition and / or locus occur from its native form through deliberate human intervention if from the same species.

"Polynucleotide", "nucleic acid sequence", "nucleotide sequence" or "nucleic acid fragment" are used interchangeably and are single-stranded or double-stranded RNA or DNA polymers, optionally containing synthetic, non-natural or altered nucleotide bases. Nucleotides are referred to by their single letter names as follows: "A" is adenosine or deoxyadenosine (corresponding to RNA or DNA, respectively), "C" means cytidine or deoxycytidine, "G" means guanosine or deoxyguanosine, "U" represents uridine, "T" means deoxythymidine, "R" means purine (A or G), "Y" means pyrimidine (C or T), "K" means G or T, "H" means A or C or T, "I" means inosine, and "N" means any nucleotide.

The codon optimization refers to a method for replacing at least one codon in the natural sequence (for example, about or more than about 1, 2, 3, 4, 5, 10, 15, 20, 25, 50 or more codons) with a codon used more frequently or most frequently in the gene of the host cell, and maintaining the natural amino acid sequence while modifying the nucleic acid sequence to enhance expression in the host cell of interest. Different species exhibit specific preferences for certain codons of specific amino acids. Codon preference (difference in codon usage between organisms) is often related to the translation efficiency of messenger RNA (mRNA), which is considered as depending on the nature of the codon being translated and the availability of the specific transfer RNA (tRNA) molecule. The advantages of the selected tRNA in the cell generally reflect the codons most frequently used for peptide synthesis. Therefore, genes may be tailored to the optimal gene expression in a given organism based on codons optimization. The codon usage tables may be easily obtained, for example, in the codon usage database ("Codon Usage Database") available at www.kazusa.orjp/codon/, and these tables may be adjusted and applied in different ways. See Nakamura Y. et al., "Codon usage tabulated from the international DNA sequence databases: status for the year 2000". Nucl. Acids Res., 28: 292 (2000).

"Polypeptide," "peptide," and "protein" are used interchangeably in the present invention to refer to a polymer of amino acid residues. The terms apply to an amino acid polymer in which one or more amino acid residues is artificial chemical analogue of corresponding naturally occurring amino acid(s), as well as to a naturally occurring amino acid polymer. The terms "polypeptide," "peptide," "amino acid sequence," and "protein" may also include modified forms including, but not limited to, glycosylation, lipid ligation, sulfation, γ carboxylation of glutamic acid residues, and ADP-ribosylation.

Sequence "identity" has recognized meaning in the art, and the percentage of sequence identity between two nucleic acids or polypeptide molecules or regions can be calculated using the disclosed techniques. Sequence identity can be measured along the entire length of a polynucleotide or polypeptide or along a region of the molecule. (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "identity" is well known to the skilled person (Carrillo, H. & Lipman, D., SIAM J Applied Math 48: 1073 (1988)).

Suitable conserved amino acid replacements in peptides or proteins are known to those skilled in the art and can generally be carried out without altering the biological activity of the resulting molecule. In general, one skilled in the art recognizes that a single amino acid replacement in a non-essential region of a polypeptide does not substantially alter biological activity (See, for example, Watson et al., Molecular Biology of the Gene, 4th Edition, 1987, The Benjamin/Cummings Pub. co., p.224).

As used in the present invention, "expression construct" refers to a vector such as a recombinant vector that is suitable for expression of a nucleotide sequence of interest in a plant. "Expression" refers to the production of a functional product. For example, expression of a nucleotide sequence may refer to the transcription of a nucleotide sequence (eg, transcription to produce mRNA or functional RNA) and / or the translation of an RNA into a precursor or mature protein.

The "expression construct" of the present invention may be a linear nucleic acid fragment, a circular plasmid, a viral vector or, in some embodiments, an RNA that is capable of translation (such as mRNA), such as an RNA generated by in vitro trascription.

The "expression construct" of the present invention may comprise regulatory sequences and nucleotide sequences of interest from different origins, or regulatory sequences and nucleotide sequences of interest from the same source but arranged in a manner different from that normally occurring in nature.

"Regulatory sequence" and "regulatory element" are used interchangeably to refer to a nucleotide sequence that is located upstream (5 'non-coding sequence), middle or downstream (3' non-coding sequence) of a coding sequence and affects the transcription, RNA processing or stability or translation of the relevant coding sequence. Regulatory sequences may include, but are not limited to, promoters, translation leaders, introns and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or tissue-specific promoter or developmentally-regulated promoter or inducible promoter.

"Constitutive promoter" refers to a promoter that may in general cause the gene to be expressed in most cases in most cell types. "Tissue-specific promoter" and "tissue-preferred promoter" are used interchangeably and mean that they are expressed primarily but not necessarily exclusively in one tissue or organ, but also in a specific cell or cell type. "Developmentally-regulated promoter" refers to a promoter whose activity is dictated by developmental events. "Inducible promoter" selectively express operably linked DNA sequences in response to an endogenous or exogenous stimulus (environment, hormones, chemical signals, etc.).

Examples of promoters include, but are not limited to, the polymerase (pol) I, pol II or pol III promoters. Promoters that can be used in plants include, but are not limited to, cauliflower mosaic virus 35S promoter, maize Ubi-1 promoter, wheat U6 promoter, rice U3 promoter, and rice actin promoter, and the like.

As used herein, the term "operably linked" refers to the linkage of a regulatory element (e.g., but not limited to, a promoter sequence, a transcription termination sequence, etc.) to a nucleic acid sequence (e.g., a coding sequence or an open reading frame) such that transcription of the nucleotide sequence is controlled and regulated by the transcriptional regulatory element. Techniques for operably linking regulatory element regions to nucleic acid molecules are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein is capable of functioning in the cell. As used in the present invention, "transformation" includes both stable and transient transformations. "Stable transformation" refers to the introduction of exogenous nucleotide sequences into the genome, resulting in the stable inheritance of foreign genes. Once stably transformed, the exogenous nucleic acid sequence is stably integrated into the genome of the organism and any of its successive generations. "Transient transformation" refers to the introduction of a nucleic acid molecule or protein into a cell, performing a function without the stable inheritance of an exogenous gene. In transient transformation, the exogenous nucleic acid sequences are not integrated into the genome.

As used herein, the term "plant" includes a whole plant and any descendant, cell, tissue, or part of a plant. The term "plant parts" include any part(s) of a plant, including, for example and without limitation: seed (including mature seed and immature seed); a plant cutting; a plant cell; a plant cell culture; a plant organ (e.g., pollen, embryos, flowers, fruits, shoots, leaves, roots, stems, and explants). A plant tissue or plant organ may be a seed, protoplast, callus, or any other group of plant cells that is organized into a structural or functional unit. A plant cell or tissue culture may be capable of regenerating a plant having the physiological and morphological characteristics of the plant from which the cell or tissue was obtained, and of regenerating a plant having substantially the same genotype as the plant. In contrast, some plant cells are not capable of being regenerated to produce plants. Regenerable cells in a plant cell or tissue culture may be embryos, protoplasts, meristematic cells, callus, pollen, leaves, anthers, roots, root tips, silk, flowers, kernels, ears, cobs, husks, or stalks.

"Progeny" of a plant comprises any subsequent generation of the plant.

### 2. Acetolactate Synthase (ALS) mutant that confers herbicide resistance

Acetolactate Synthase (ALS) is a key enzyme in the synthesis of branched-chain amino acids in plants, microorganisms and fungi. Branched-chain amino acids, including valine, leucine and isoleucine, are essential amino acids for protein synthesis and plant growth in plants. The biosynthetic pathway of branched-chain amino acids requires the participation of four enzymes. Among them, acetolactate synthase is responsible for catalyzing 2 molecules of pyruvate to generate acetolactate and carbon dioxide in the first step of the synthesis of valine and leucine. During the synthesis of leucine, it is responsible for catalyzing 1 molecule of pyruvate and 1 molecule of α-butyric acid to generate 2-acetaldehyde-2-hydroxybutyric acid and carbon dioxide. If ALS activity is inhibited or destroyed, it will lead to the lack of three essential amino acids, which will affect the normal growth of plants.

ALS-inhibiting herbicides include, but are not limited to, sulfonylureas (SU), imidazolinones (IMI), triazolopyrimidines (TP), pyrimidinylthiobenzoates (PTB), and sulfonamido-carbonyls - Triazolinones (SCT) compounds, or any combination thereof. Suitable sulfonylurea compounds include, but are not limited to, chlorsulfuron-methyl, flusulfuron-methyl, trisulfuron-methyl, metsulfuron-methyl, chlorsulfuron-methyl, trifensulfuron-methyl, metsulfuron-methyl, thifensulfuron-methyl, pyrazosulfuron-methyl, Amisulfuron, Nicosulfuron, etc. Examples of suitable imidazolinones include, but are not limited to, imazethapyr, imazapic, imazaquin, and the like. Other suitable ALS inhibitors include, but are not limited to, penoxsulam, foxsulfuron, bispyribac, and the like.

In the present invention, the inventors created and identified novel ALS mutants that are resistant to ALS-inhibiting herbicides through base editing techniques.

Therefore, in one aspect, the present invention provides a Acetolactate Synthase (ALS) mutant or a functional fragment thereof, which has an amino acid mutation at one or more positions selected from position 99, 98, and 102 as compared to wild-type ALS, with the ALS amino acid positions referring to SEQ ID NO:1.

As used herein, "amino acid position refers/referring to SEQ ID NO: x" (SEQ ID NO: x is a specific sequence listed herein) means that the position number of the specific amino acid described is the position number of the amino acid in SEQ ID NO: x to which that the described amino acid corresponds to. The correspondence of amino acids in different sequences can be determined according to sequence alignment methods known in the art. For example, the amino acid correspondence can be determined by the online comparison tool of EMBL-EBI (https://www.ebi.ac.uk/Tools/psa/), and the two sequences can use the Needleman-Wunsch algorithm, using the default parameters to align. For example, the alanine at position 98 from the N-terminus in the wild-type ALS encoded by the wheat B genome is aligned with the amino acid at position 99 of SEQ ID NO: 1 (wild-type ALS encoded by the wheat A genome) in a sequence alignment, the alanine can be described herein as "the alanine at position 99 of the wild-type ALS encoded by the wheat B genome, the ALS amino acid position referring to SEQ ID NO: 1".

In some embodiments, the ALS mutant or functional fragment thereof, when expressed in a plant, can confer resistance to herbicides to the plant.

In some embodiments, the ALS mutant is derived from wheat wild-type ALS. In some embodiments, the wheat wild-type ALS is the wild-type ALS encoded by the wheat A genome. An exemplary wheat A genome-encoded wild-type ALS comprises the amino acid sequence set forth in SEQ ID NO:1. In some embodiments, the wheat wild-type ALS is the wild-type ALS encoded by the wheat B genome. An exemplary wheat B genome-encoded wild-type ALS comprises the amino acid sequence set forth in SEQ ID NO:2. In some embodiments, the wheat wild-type ALS is the wild-type ALS encoded by the wheat D genome. An exemplary wheat D genome-encoded wild-type ALS comprises the amino acid sequence set forth in SEQ ID NO:3.

In some embodiments, the alanine (A) at position 99 of the ALS mutant or functional fragment thereof is substituted relative to wild-type ALS, the ALS amino acid position referring to SEQ ID NO:1. In some embodiments, the glycine (G) at position 98 of the ALS mutant or functional fragment thereof is substituted relative to wild-type ALS, the ALS amino acid position referring to SEQ ID NO:1. In some embodiments, the methionine (M) at position 101 of the ALS mutant or functional fragment thereof is substituted relative to wild-type ALS, the ALS amino acid position referring to SEQ ID NO:1. In some embodiments, the glutamic acid (E) at position 102 of the ALS mutant or functional fragment thereof is substituted relative to wild-type ALS, the ALS amino acid position referring to SEQ ID NO:1. In some embodiments, the alanine (A)at position 99 and glycine (G) at position 98 of the ALS mutant or functional fragment thereof are substituted relative to wild-type ALS, the ALS amino acid position referring to SEQ ID NO:1. In some embodiments, alanine (A) at position 99 of the ALS mutant or functional fragment thereof is substituted relative to wild-type ALS, the ALS amino acid position referring to SEQ ID NO:1. In some embodiments, alanine (A) at position 99, glycine (G) at position 98, methionine (M) at position 101 and glutamic acid (E) at position 102of the ALS mutant or functional fragment thereof are substituted relative to wild-type ALS, the ALS amino acid position referring to SEQ ID NO:1

In some embodiments, the alanine (A) at position 99 of the ALS mutant or functional fragment thereof is substituted by a threonine (T) relative to wild-type ALS, the ALS amino acid position referring to SEQ ID NO:1. In some embodiments, the glycine (G) at position 98 of the ALS mutant or functional fragment thereof is substituted by an asparagine (N)relative to wild-type ALS, the ALS amino acid position referring to SEQ ID NO:1. In some embodiments, the methionine (M) at position 101 of the ALS mutant or functional fragment thereof is substituted by an isoleucine (I) relative to wild-type ALS, the ALS amino acid position referring to SEQ ID NO:1. In some embodiments, the glutamic acid (E) at position 102 of the ALS mutant or functional fragment thereof is substituted by a lysine (K) relative to wild-type ALS, the ALS amino acid position referring to SEQ ID NO:1. In some embodiments, the alanine (A) at position 99 of the ALS mutant or functional fragment thereof is substituted by a threonine (T) and the glycine at position 98 ( G) is substituted by an asparagine (N) relative to wild-type ALS, the ALS amino acid position referring to SEQ ID NO:1. In some embodiments, alanine (A) at position 99 of the ALS mutant or functional fragment thereof is substituted by a threonine (T), glycine (G) at position 98 is substituted by asparagine (N), methionine (M) at position 101 is substituted by isoleucine (I) and glutamic acid (E) at position 102 is substituted by lysine (K)relative to wild-type ALS, the ALS amino acid position referring to SEQ ID NO:1.

In some embodiments, the ALS mutant or functional fragment thereof comprises one or more amino acid substitutions selected from the group consisting of A99T, G98N, M101I, E102K relative to wild-type ALS, the ALS amino acid positions referring to SEQ ID NO: 1.

As used herein, a "functional fragment" of an ALS mutant refers to a fragment that at least partially or fully retains the function of the full-length ALS mutant from which it is derived.

In some embodiments, the ALS mutant or functional fragment thereof further comprises one or more additional amino acid mutations, e.g., conservative amino acid substitutions, relative to wild-type ALS.

In some embodiments, the ALS mutant or functional fragment thereof comprises an amino acid sequence having at least 80%, at least 90%, at least 91%, at least 92%, at least 93%, at least 94%, at least 95%, at least 96%, at least 97%, at least 98%, at least 99%, at least 99.5%, even 100% sequence identity to any one of SEQ ID NOs:4-8.

### 3. Protoporphyrinogen oxidase (PPO) mutant that confers herbicide resistance

Protoporphyrinogen oxidase (PPO) catalyzes the reaction of protoporphyrinogen IX to form protoporphyrin IX in the synthesis of chlorophyll, and is a key enzyme in the synthesis of chlorophyll. PPO is located in the chloroplast, and the inhibition of this enzyme will cause the rapid accumulation of light-sensitive protoporphyrinogen IX, which penetrates the chloroplast membrane and penetrates into the cytoplasm outside the chloroplast. In the cytoplasm, protoporphyrinogen IX is automatically oxidized to protoporphyrin IX, and high concentration of protoporphyrin IX will generate toxic singlet oxygen under the action of light and oxygen, thereby causing the peroxidation of unsaturated fatty acids in the cell membrane, resulting in membranes leaking, pigments destroyed, and eventually leaves died.

PPO-inhibiting herbicides include, but are not limited to, diphenyl ethers, cyclic imines, phenylpyrazoles, bicyclic triazolones, or any combination thereof. Suitable PPO-inhibiting herbicides include, but are not limited to, acifluorfen, fenoxafen, fenflufen, fenflufen-prop, fenflufen-ethyl, flufenox, fenflufenac, fenflufenac Amyl Ester, Profenazone, Acifluorfen, Methoxamate, Fomesafen, Lactofen, Oxadiazone, Oxadiazon, Oxaflufen, Metazapyr , Sulfentrazone, Sulfentrazone, Caroxazone, etc.

In the present invention, the inventors created and identified novel PPO mutants that are resistant to PPO-inhibiting herbicides through base editing technology.

Therefore, in one aspect, the present invention provides a Protoporphyrinogen oxidase (PPO) mutant or a functional fragment thereof, which has an amino acid mutation at one or more positions selected from position 127 and 129 as compared to wild-type ALS, with the PPO amino acid positions referring to SEQ ID NO:9.

In some embodiments, the PPO mutant or functional fragment thereof, when expressed in a plant, can confer resistance to herbicides to the plant.

In some embodiments, the PPO mutant is derived from wheat wild-type PPO. In some embodiments, the wheat wild-type PPO is the wild-type PPO encoded by the wheat A genome. An exemplary wheat A genome-encoded wild-type PPO comprises the amino acid sequence set forth in SEQ ID NO:9. In some embodiments, the wheat wild-type PPO is the wild-type PPO encoded by the wheat B genome. An exemplary wheat B genome-encoded wild-type PPO comprises the amino acid sequence set forth in SEQ ID NO:10. In some embodiments, the wheat wild-type PPO is the wild-type PPO encoded by the wheat D genome. An exemplary wheat D genome-encoded wild-type PPO comprises the amino acid sequence set forth in SEQ ID NO: 11.

In some embodiments, the arginine (R) at position 127 of the PPO mutant or functional fragment thereof is substituted relative to wild-type PPO, the PPO amino acid position referring to SEQ ID NO:9. In some embodiments, the threonine (T) at position 129the PPO mutant or functional fragment thereof is substituted relative to wild-type PPO, the PPO amino acid position referring to SEQ ID NO:9. In some embodiments, arginine (R) at position 127 and threonine (T) at position 129 of the PPO mutant or functional fragment thereof are substituted relative to wild-type PPO, the PPO amino acid position referring to SEQ ID NO:9.

In some embodiments, the arginine (R) at position 127 of the PPO mutant or functional fragment thereof is substituted by cysteine (C) or glycine (G) relative to wild-type PPO, the PPO amino acid position referring to SEQ ID NO:9. In some embodiments, the threonine (T) at position 129 the PPO mutant or functional fragment thereof is substituted by an isoleucine (I) relative to wild-type PPO, the PPO amino acid position referring to SEQ ID NO:9. In some embodiments, arginine (R) at position 127 of the PPO mutant or functional fragment thereof is substituted by cysteine (C) or glycine (G), and threonine (T) at position 129 is substituted by an isoleucine (I) relative to wild-type PPO, the PPO amino acid position referring to SEQ ID NO:9.

In some embodiments, the PPO mutant or functional fragment thereof comprises one or more amino acid substitutions selected from the group consisting of R127C, R127G, T129I relative to wild-type PPO, the PPO amino acid position referring to SEQ ID NO:9.

As used herein, a "functional fragment" of an PPO mutant refers to a fragment that at least partially or fully retains the function of the full-length PPO mutant from which it is derived.

In some embodiments, the PPO mutant or functional fragment thereof further comprises one or more additional amino acid mutations, e.g., conservative amino acid substitutions, relative to wild-type PPO.

In some embodiments, the PPO mutant or functional fragment thereof comprises an amino acid sequence having at least 80%, at least 90%, at least 91%, at least 92%, at least 93%, at least 94%, at least 95%, at least 96%, at least 97%, at least 98%, at least 99%, at least 99.5%, even 100% sequence identity to any one of SEQ ID NOs: 12-16.

### 4. Acetyl-CoA carboxylase (ACCase) mutant that confers herbicide resistance

Acetyl-CoA carboxylase (ACCase) is a biotinylase that catalyzes the carboxylation of acetyl-CoA to generate malonyl-CoA. This carboxylation is a two-step reversible reaction consisting of ATP-dependent carboxylation of the biotin group of the carboxyl carrier domain by biotin carboxylase activity, followed by carboxytransferase activity of the carboxyl group transferred from biotin to acetyl-CoA (Nikolau et al., 2003, Arch. Biochem. Biophys. 414:211-22). Acetyl-CoA carboxylase is not only a key enzyme in fatty acid synthesis in plants (synthesis occurs in chloroplasts and mitochondria), but is also involved in the formation of long-chain fatty acids and flavonoids, as well as malonylation that occurs in the cytoplasm function in the process. There are two isoforms of ACCase, of which chloroplast ACCase accounts for more than 80% of total ACCase activity (Herbert et al., 1996, Biochem. J. 318:997-1006). Aryloxyphenoxypropionates (FOPs) and cyclohexanediones (DIMs) are two classes of chemicals known to selectively inhibit chloroplast ACCase in weeds (Rendina et al., 1990, J.Agric. Food Chem. 38: 1282-1287). In addition, phenylpyrazolines (DENs) can also inhibit ACCase activity.

In the present invention, the inventors created and identified novel ACCase mutants that are resistant to herbicides such as aryloxyphenoxypropionates (FOPs), cyclohexanediones (DIMs) or phenylpyrazolines (DEN) through base editing techniques.

Therefore, in one aspect, the present invention provides a ACCase mutant or a functional fragment thereof, which has an amino acid mutation at one or more positions selected from position 1866, 1884, 1927 or 2181 as compared to wild-type ALS, with the ACCase amino acid positions referring to SEQ ID NO:31.

In some embodiments, the ACCase mutant or functional fragment thereof, when expressed in a plant, can confer resistance to herbicides to the plant.

The herbicides of various embodiments of the present invention include herbicides capable of inhibiting ACCase activity (ACCase inhibitors), such as aryloxyphenoxypropionates, cyclohexanediones, or phenylpyrazolines, or any combination of them. Examples of suitable aryloxyphenoxypropionate herbicides include, but are not limited to, clodinafop-propargyl, cyhalofop-butyl, diclofop-methyl, fenoxaprop-p-ethyl, fluazifop-b-butyl, haloxyfop-ethoxyethyl, haloxyfop-etotyl, haloxyfop-etotyl Haloxyfop-R-methyl, propaquizafop, quizalofop-p-ethyl, haloxyfop-P, or any combination thereof. Examples of suitable cyclohexanedione herbicides include, but are not limited to, alloxydim, butroxydim, clefoxydim, clethodim, cycloxydim, cycloxydim (profoxydim), sethoxydim, teproloxydim, and tralkoxydim, or any combination thereof. Examples of suitable phenylpyrazoline herbicides include, but are not limited to, pinoxaden.

In some embodiments, the ACCase mutant is derived from a rice wild-type ACCase. An exemplary rice wild-type ACCase comprises the amino acid sequence set forth in SEQ ID NO:31.

In some embodiments, the serine (S) at position 1866 of the ACCase mutant or functional fragment thereof is substituted relative to the wild-type ACCase, the ACCase amino acid position referring to SEQ ID NO:31. In some embodiments, the alanine (A) at position 1884 of the ACCase mutant or functional fragment thereof is substituted relative to the wild-type ACCase, the ACCase amino acid position referring to SEQ ID NO:31. In some embodiments, the proline (P) at position 1927 of the ACCase mutant or functional fragment thereof is substituted relative to the wild-type ACCase, the ACCase amino acid position referring to SEQ ID NO:31. In some embodiments, the proline (P) at position 2181 of the ACCase mutant or functional fragment thereof is substituted relative to the wild-type ACCase, and the ACCase amino acid position referring to SEQ ID NO:31.

In some embodiments, the serine (S) at position 1866 of the ACCase mutant or functional fragment thereof is substituted by a phenylalanine (F) relative to the wild-type ACCase, the ACCase amino acid position referring to SEQ ID NO:31. In some embodiments, the alanine (A) at position 1884 of the ACCase mutant or functional fragment thereof is substituted with a proline (P) relative to the wild-type ACCase, the ACCase amino acid position referring to SEQ ID NO:31. In some embodiments, the proline (P) at position 1927 of the ACCase mutant or functional fragment thereof is substituted with a phenylalanine (F) relative to the wild-type ACCase, the ACCase amino acid position referring to SEQ ID NO:31. In some embodiments, the proline (P) at position 2181 of the ACCase mutant or functional fragment thereof is substituted with a leucine (L) relative to the wild-type ACCase, and the ACCase amino acid position referring to SEQ ID NO:31.

In some embodiments, the ACCase mutant or functional fragment thereof comprises one or more amino acid substitutions selected from the group consisting of S1866F, A1884P, P1927, P2181L relative to wild-type ACCase, the ACCase amino acid positions referring to SEQ ID NO: 31.

As used herein, a "functional fragment" of an ACCase mutant refers to a fragment that at least partially or fully retains the function of the full-length ACCase mutant from which it is derived. For example, the functional fragment comprises at least the carboxyltransferase (CT) domain of ACCase and comprises the amino acid mutations described above. The amino acid sequence of an exemplary wild-type ACCase carboxyltransferase (CT) domain is set forth in SEQ ID NO:36.

In some embodiments, the ACCase mutant or functional fragment thereof further comprises one or more additional amino acid mutations, e.g., conservative amino acid substitutions, relative to wild-type ACCase. Preferably, the additional amino acid mutation is located outside the CT domain.

In some embodiments, the ACCase mutant or functional fragment thereof comprises an amino acid sequence of any one of SEQ ID NOs:32-35 or 37-40. In some embodiments, the ACCase mutant or functional fragment thereof comprises an amino acid sequence having at least 80%, at least 90%, at least 91%, at least 92%, at least 93%, at least 94%, at least 95%, at least 96%, at least 97%, at least 98%, at least 99%, at least 99.5% sequence identity to any one of SEQ ID NOs:32-35 or 37-40.

### 5. p-hydroxyphenylpyruvate dioxidase (HPPD) mutant that confers herbicide resistance

P-Hydroxyphenylpyruvate dioxidase (HPPD) participates in two metabolic pathways, tyrosine catabolism and plastoquinone and tocopherol biosynthesis. P-Hydroxyphenylpyruvate is converted to homogentisic acid under the catalysis of HPPD. Homogentisic acid is further decarboxylated and alkylated to produce plastoquinone and tocopherol. Plastoquinone acts as the ultimate electron acceptor and electron transporter in the photosynthetic chain in carotenoid biosynthesis. The lack of plastoquinone in the thylakoid will lead to a decrease in carotenoid biosynthesis. P-hydroxyphenylpyruvate dioxidase (HPPD) inhibitors have broad-spectrum herbicidal activity, can control broad-leaved weeds in broad-leaved crops, can be used before or after emergence, with high activity, low residue, good environmental compatibility and safety.

HPPD inhibitory herbicides include, but are not limited to, pyrazoles, triketones, isoxazoles, diketonitriles, and benzophenone compounds, or any combinations thereof. Suitable pyrazole compounds include, but are not limited to, mefentrazone (Bauwei), sulfenylpyrazole, phenazopyramide and the like. Suitable triketone compounds include, but are not limited to, sulcotrione, mesotrione, terbotrione, tetrafuryl ketone, bicyclopyrone, benzobicycloketone, and the like. Suitable isoxazole compounds include, but are not limited to, isoxaflutole. Suitable diketonitrile compounds include, but are not limited to, 2-cyano-3-cyclopropyl-1-(2-methylsulfonyl-4-trifluoromethylphenyl)-propane-1,3-dione and 2-cyano-1-[4-(methylsulfonyl)-2-trifluoromethylphenyl]-3-(1-methylcyclopropyl)propane-1,3 -dione.

In the present invention, the inventors created and identified novel HPPD mutants that are resistant to HPPD-inhibiting herbicides through base editing technology.

Therefore, in one aspect, the present invention provides a HPPD mutant or a functional fragment thereof, which has an amino acid mutation at one or more positions selected from position 365, 379, 353, 354, 346, 347, 369 or 370 as compared to wild-type HPPD, with the HPPD amino acid positions referring to SEQ ID NO:45.

As used herein, "amino acid position refers/referring to SEQ ID NO: x" (SEQ ID NO: x is a specific sequence listed herein) means that the position number of the specific amino acid described is the position number of the amino acid in SEQ ID NO: x to which that the described amino acid corresponds to. The correspondence of amino acids in different sequences can be determined according to sequence alignment methods known in the art. For example, the amino acid correspondence can be determined by the online comparison tool of EMBL-EBI (https://www.ebi.ac.uk/Tools/psa/), and the two sequences can use the Needleman-Wunsch algorithm, using the default parameters to align.

For example, the amino acid at position 360 from the N-terminus in one polypeptide is aligned with the amino acid at position 365 of SEQ ID NO: x in a sequence alignment, said amino acid in said polypeptide can be described herein as "the alanine at position 365 of the polypeptide, the amino acid position referring to SEQ ID NO: x".

In some embodiments, the HPPD mutant or functional fragment thereof, when expressed in a plant, can confer resistance to herbicides to the plant.

In some embodiments, the HPPD mutant is derived from a rice wild-type HPPD. An exemplary rice wild-type HPPD comprises the amino acid sequence set forth in SEQ ID NO:45.

In some embodiments, the leucine (L) at position 365 of the HPPD mutant or functional fragment thereof is substituted relative to wild-type HPPD, the HPPD amino acid position referring to SEQ ID NO:45. In some embodiments, the threonine (T) at position 379 of the HPPD mutant or functional fragment thereof is substituted relative to wild-type HPPD, the HPPD amino acid position referring to SEQ ID NO:45. In some embodiments, the glutamic acid (E) at position 353 of the HPPD mutant or functional fragment thereof is substituted relative to wild-type HPPD, the HPPD amino acid position referring to SEQ ID NO:45. In some embodiments, the glutamic acid (E) at position 354 of the HPPD mutant or functional fragment thereof is substituted relative to wild-type HPPD, the HPPD amino acid position referring to SEQ ID NO:45. In some embodiments, the glutamic acid (E) at position 353 and glutamic acid (E) at position 354 of the HPPD mutant or functional fragment thereof are substituted relative to wild-type HPPD, the HPPD amino acid positions referring to SEQ ID NO:45. In some embodiments, the arginine (R) at position 346 of the HPPD mutant or functional fragment thereof is substituted relative to wild-type HPPD, the HPPD amino acid position referring to SEQ ID NO:45. In some embodiments, the alanine (A) at position 347 of the HPPD mutant or functional fragment thereof is substituted relative to wild-type HPPD, the HPPD amino acid position referring to SEQ ID NO:45. In some embodiments, the arginine (R) at position 346 and alanine (A) at position 347 of the HPPD mutant or functional fragment thereof is substituted relative to wild-type HPPD, the HPPD amino acid positions referring to SEQ ID NO:45. In some embodiments, the aspartic acid (D) at position 369 of the HPPD mutant or functional fragment thereof is substituted relative to wild-type HPPD, the HPPD amino acid position referring to SEQ ID NO:45. In some embodiments, the aspartic acid (D) at position 370 of the HPPD mutant or functional fragment thereof is substituted relative to wild-type HPPD, the HPPD amino acid position referring to SEQ ID NO:45. In some embodiments, the aspartic acid (D) at position 369 and aspartic acid (D) at position 370 of the HPPD mutant or functional fragment thereof is substituted relative to wild-type HPPD, The HPPD amino acid positions are referenced to SEQ ID NO:45.

In some embodiments, the leucine (L) at position 365 of the HPPD mutant or functional fragment thereof is substituted by a Phenylalanine (F) relative to wild-type HPPD, the HPPD amino acid position referring to SEQ ID NO:45. In some embodiments, the threonine (T) at position 379 of the HPPD mutant or functional fragment thereof is substituted by an Isoleucine (I) relative to wild-type HPPD, the HPPD amino acid position referring to SEQ ID NO:45. In some embodiments, the glutamic acid (E) at position 353 of the HPPD mutant or functional fragment thereof is substituted by a Lysine (K) relative to wild-type HPPD, the HPPD amino acid position referring to SEQ ID NO:45. In some embodiments, the glutamic acid (E) at position 354 of the HPPD mutant or functional fragment thereof is substituted by a Lysine (K) relative to wild-type HPPD, the HPPD amino acid position referring to SEQ ID NO:45. In some embodiments, the glutamic acid (E) at position 353 and glutamic acid (E) at position 354 of the HPPD mutant or functional fragment thereof are substituted by a Lysine (K) respectively relative to wild-type HPPD, the HPPD amino acid positions referring to SEQ ID NO:45. In some embodiments, the arginine (R) at position 346 of the HPPD mutant or functional fragment thereof is substituted by a Cysteine (C) relative to wild-type HPPD, the HPPD amino acid position referring to SEQ ID NO:45. In some embodiments, the alanine (A) at position 347 of the HPPD mutant or functional fragment thereof is substituted by a Valine (V) relative to wild-type HPPD, the HPPD amino acid position referring to SEQ ID NO:45. In some embodiments, the arginine (R) at position 346 of the HPPD mutant or functional fragment thereof is substituted by a Cysteine (C) and the alanine (A) at position 347 of the HPPD mutant or functional fragment thereof is substituted by a Valine (V) relative to wild-type HPPD, the HPPD amino acid positions referring to SEQ ID NO:45. In some embodiments, the aspartic acid (D) at position 369 of the HPPD mutant or functional fragment thereof is substituted by an Asparagine (N) relative to wild-type HPPD, the HPPD amino acid position referring to SEQ ID NO:45. In some embodiments, the aspartic acid (D) at position 370 of the HPPD mutant or functional fragment thereof is substituted an Asparagine (N) relative to wild-type HPPD, the HPPD amino acid position referring to SEQ ID NO:45. In some embodiments, the aspartic acid (D) at position 369 and aspartic acid (D) at position 370 of the HPPD mutant or functional fragment thereof are substituted an Asparagine (N) respectively relative to wild-type HPPD, The HPPD amino acid positions are referenced to SEQ ID NO:45.

In some embodiments, the HPPD mutant or functional fragment thereof comprises one or more amino acid substitutions selected from the group consisting of L365F, T379I, E353K, E354K, E353K/E354K, R346C, A347V, R346C/A347V, D369N, D370N, D369N/D370N relative to wild-type HPPD, the HPPD amino acid positions referring to SEQ ID NO: 45.

As used herein, a "functional fragment" of an HPPDmutant refers to a fragment that at least partially or fully retains the function of the full-length HPPDmutant from which it is derived.

In some embodiments, the HPPDmutant or functional fragment thereof further comprises one or more additional amino acid mutations, e.g., conservative amino acid substitutions, relative to wild-type HPPD.

In some embodiments, the HPPD mutant or functional fragment thereof comprises an amino acid sequence having at least 80%, at least 90%, at least 91%, at least 92%, at least 93%, at least 94%, at least 95%, at least 96%, at least 97%, at least 98%, at least 99%, at least 99.5% sequence identity to any one of SEQ ID NOs:46-50. In some embodiments, the HPPD mutant comprises the amino acid sequence set forth in any of SEQ ID NOs: 46-50.

### 6. Related Nucleic Acids, Constructs and Herbicide Resistant Plants

In another aspect, the present invention also provides a nucleic acid comprising a nucleotide sequence encoding the ALS mutant of the present invention or a functional fragment thereof, and/or a nucleotide sequence encoding the PPO mutant of the present invention or a functional fragment thereof, and/or a nucleotide sequence encoding the ACCase mutants of the present invention or a functional fragment thereof, and/or a nucleotide sequence encoding the HPPD mutants of the present invention or a functional fragment thereof. In some embodiments, the nucleic acid is an isolated nucleic acid or a recombinant nucleic acid.

In some embodiments, the nucleotide sequence encoding the ALS mutant of the present invention or a functional fragment thereof, and/or the nucleotide sequence encoding the PPO mutant of the present invention or a functional fragment thereof, and/or the nucleotide sequence encoding the ACCase mutant of the present invention or a functional fragment thereof, and/or the nucleotide sequence encoding the HPPD mutant of the present invention or a functional fragment thereof can be codon-optimized against the plant of interest.

In another aspect, the present invention also provides an expression cassette comprising a nucleotide sequence encoding the ALS mutant of the present invention or a functional fragment thereof, and/or a nucleotide sequence encoding the PPO mutant of the present invention or a functional fragment thereof, and/or a nucleotide sequence encoding the ACCase mutants of the present invention or a functional fragment thereof, and/or a nucleotide sequence encoding the HPPD mutants of the present invention or a functional fragment thereof, operably linked to a regulatory sequence.

In another aspect, the present invention also provides an expression construct comprising the expression cassette of the present invention.

In another aspect, the present invention also provides the use of the ALS mutant of the present invention or a functional fragment thereof, the PPO mutant of the present invention or a functional fragment thereof, the ACCase mutant of the present invention or a functional fragment thereof, the HPPD mutations of the present invention or a functional fragment thereof, the isolated nucleic acid of the present invention, the expression cassette of the present invention, or the expression construct of the present invention in the production of a herbicide-resistant plant.

In another aspect, the present invention also provides a herbicide-resistant plant comprising (e.g. expressing) the ALS mutant of the present invention or a functional fragment thereof, and/or the PPO mutant of the present invention or a functional fragment thereof, and/or the ACCase mutant of the present invention or a functional fragment thereof, and/or the HPPD mutant of the present invention or a functional fragment thereof.

The plants described in various aspects of the invention may be plants that are susceptible to ALS inhibitors and/or PPO inhibitors and/or ACCase inhibitors and/or HPPD inhibitors, including monocotyledonous or dicotyledonous plants. Preferably, the plant is a crop plant, such as a monocotyledonous crop plant. Examples of suitable plants include, but are not limited to, rice, wheat, barley, sorghum, corn, and the like. In some preferred embodiments, the plant is wheat. In some preferred embodiments, the plant is rice.

The herbicide resistance in various aspects of the invention may be resistance to an ALS-inhibiting herbicide and/or a PPO-inhibiting herbicide and/or an ACCase-inhibiting herbicide and/or a HPPD-inhibiting herbicide.

### 7. Methods of producing herbicide-resistant plants by transgene

In another aspect, the present invention also provides a method for production of herbicide-resistant plants through transgene, comprising introducing the nucleic acid of the present invention, the expression cassette of the present invention and/or the expression construct of the present invention into a plant.

Various methods known in the art can be used to introduce the nucleic acid of the present invention, the expression cassette of the present invention, and/or the expression construct of the present invention into the plant. Suitable introduction methods include, but are not limited to, gene bombardment, PEG-mediated transformation of protoplasts, Agrobacterium-mediated transformation, plant virus-mediated transformation, pollen tube approach, and ovary injection approach.

In some embodiments, the nucleic acid of the invention, the expression cassette of the invention and/or the expression construct of the invention are integrated into the genome of the plant. The isolated nucleic acid of the present invention, the expression cassette of the present invention and/or the expression construct of the present invention will confer to said plants resistance to herbicides capable of inhibiting ALS activity and/or PPO activity and/or ACCase activity and/or HPPD activity.

In another aspect, the present invention also provides a herbicide-resistant plant which comprises the expression cassette of the present invention, the nucleic acid or the expression construct of the present invention, or is transformed with the nucleic acid of the present invention, the expression cassette of the present invention and/or the expression construct of the present invention. The present invention also encompasses the progeny of said herbicide-resistant plant.

The plants described in various aspects of the invention may be plants that are susceptible to ALS inhibitors and/or PPO inhibitors and/or ACCase inhibitors and/or HPPD inhibitors, including monocotyledonous or dicotyledonous plants. Preferably, the plant is a crop plant, such as a monocotyledonous crop plant. Examples of suitable plants include, but are not limited to, rice, wheat, barley, sorghum, corn, and the like. In some preferred embodiments, the plant is wheat. In some preferred embodiments, the plant is rice.

### 8. Methods for generating herbicide-resistant plants by base editing

In another aspect, the present invention also provides a method for generating a herbicide-resistant plant by base editing, comprising introducing into the plant a base editing system targeting an endogenous ALS coding region and/or an endogenous PPO coding region and/or an endogenous ACCase coding region and/or an endogenous HPPD coding region in the genome of the plant, thereby resulting in
(i) amino acid mutation in the endogenous ALS at one or more positions selected from the group consisting of positions 99, 98, 101 and 102, said ALS amino acid position referring to SEQ ID NO: 1, and/or
(ii) amino acid mutation in the endogenous PPO at one or more positions selected from the group consisting of positions 127 and 129, the PPO amino acid positions referring to SEQ ID NO: 9, and/or
(iii) amino acid mutation in the endogenous ACCase at one or more positions selected from the group consisting of positions 1866, 1884, 1927 or 2181, said ACCase amino acid positionsreferring to SEQ ID NO: 31, and/or
(iv) amino acid mutation in the endogenous HPPD at one or more positions selected from the group consisting of positions 365, 379, 353, 354, 346, 347, 369 or 370, the HPPD amino acid position referring to SEQ ID NO:45.

In some embodiments, introduction of the base editing system results in that the alanine (A) at position 99 and/or the glycine (G) at position 98 and/or the methionine (M) at position 101 and/or glutamic acid (E) at position 102 in the endogenous ALS is substituted, with the ALS amino acid position referring to SEQ ID NO:1.

In some embodiments, introduction of the base editing system results in that the alanine (A) at position 99 is substituted with a threonine (T) and/or the glycine (G) at position 98 is substituted with asparagine (N) and/or the methionine (M) at position 101 is substituted with isoleucine (I) and/or glutamic acid (E) at position 102 in the endogenous ALS is substituted by lysine (K), with the ALS amino acid position referring to SEQ ID NO:1.

In some embodiments, introduction of the base editing system results in the endogenous ALS comprising one or more amino acid substitutions selected from the group consisting of A99T, G98N, M101I, E102K, the ALS amino acid positions referring to SEQ ID NO:1.

In some embodiments, introduction of the base editing system results in that the arginine (R) at position 127 and/or the threonine (T) at position 129 of the endogenous PPO is substituted, the PPO amino acid position referring to SEQ ID NO:9.

In some embodiments, introduction of the base editing system results in that the arginine (R) at position 127 is substituted with a cysteine (C) or a glycine (G) and/or the threonine (T) at position 129 of the endogenous PPO is substituted with isoleucine (I), the PPO amino acid position referring to SEQ ID NO:9.

In some embodiments, introduction of the base editing system results in the endogenous PPO comprising one or more amino acid substitutions selected from the group consisting of R127C, R127G, T129I, the PPO amino acid positions referring to SEQ ID NO:9.

In some embodiments, introduction of the base editing system results in that in the endogenous ACCase, the serine (S) at position 1866 is substituted, and/or the alanine (A) at position 1884 is substituted, and/or the proline (P) at position 1927 is substituted, and/or the proline (P) at position 2181 is substituted, the ACCase amino acid position referring to SEQ ID NO:31.

In some embodiments, introduction of the base editing system results in that in the endogenous ACCase, the serine (S) at position 1866 is substituted by phenylalanine (F), and/or the alanine (A) at position 1884 is substituted with proline (P), and/or the proline (P) at position 1927 is substituted with phenylalanine (F), and/or the proline (P) at position 2181 is substituted with leucine (L), the ACCase amino acid position referring to SEQ ID NO:31.

In some embodiments, introduction of the base editing system results in the endogenous ACCase comprising one or more amino acid substitutions selected from the group consisting of S1866F, A1884P, P1927, P2181L, the ACCase amino acid positions referring to SEQ ID NO:31.

In some embodiments, introduction of the base editing system results in that the leucine (L) at position 365 and/or the threonine (T) at position 379, and/or the glutamic acid (E) at position 353, and/or the glutamic acid (E) at position 354, and/or the arginine (R) at position 346, and/or the alanine (A) at position 347, and/or the aspartic acid (D) at position 369, and/or the aspartic acid (D) at position 370 of the endogenous HPPD is substituted, with the HPPD amino acid positions referring to SEQ ID NO:45.

In some embodiments, introduction of the base editing system results in that the leucine (L) at position 365 is substituted by Phenylalanine (F) and/or the threonine (T) at position 379 is substituted by Isoleucine (I), and/or the glutamic acid (E) at position 353 is substituted by lysine (K), and/or the glutamic acid (E) at position 354 is substituted by lysine (K), and/or the arginine (R) at position 346 is substituted by cysteine (C), and/or the alanine (A) at position 347 is substituted by Valine (V), and/or the aspartic acid (D) at position 369 is substituted by Asparagine (N), and/or the aspartic acid (D) at position 370 of the endogenous HPPD is substituted by Asparagine (N), with the HPPD amino acid positions referring to SEQ ID NO:45.

In some embodiments, introduction of the base editing system results in the endogenous HPPD comprising one or more amino acid substitutions selected from the group consisting of L365F, T379I, E353K, E354K, E353K/E354K, R346C, A347V, R346C/A347V, D369N, D370N, D369N/D370N, the HPPD amino acid positions referring to SEQ ID NO:45.

The base editing system that can be used in the present invention may be various base editing systems known in the art as long as it can perform targeted genome base editing in plants. For example, such base editing systems include, but are not limited to, those described in WO 2018/056623, WO 2019/120283, WO 2019/120310.

In some embodiments, the base editing system comprises a base editing fusion protein or an expression construct comprising a nucleotide sequence encoding the base editing fusion protein, and at least one guide RNA or an expression construct comprising a nucleotide sequence encoding at least one guide RNA, for example, the system includes at least one of the following i) to v):
i) a base editing fusion protein, and at least one guide RNA;
ii) an expression construct comprising a nucleotide sequence encoding a base editing fusion protein, and at least one guide RNA;
iii) a base editing fusion protein, and an expression construct containing a nucleotide sequence encoding at least one guide RNA;
iv) an expression construct comprising a nucleotide sequence encoding a base editing fusion protein, and an expression construct comprising a nucleotide sequence encoding at least one guide RNA;
v) an expression construct comprising a nucleotide sequence encoding a base editing fusion protein and a nucleotide sequence encoding at least one guide RNA;
   wherein the base editing fusion protein comprises a CRISPR effector protein and a deaminase domain, and the at least one guide RNA can target the base editing fusion protein to an endogenous ALS coding sequence and/or an endogenous PPO coding sequence and/or an endogenous ACCase coding sequence and/or an endogenous HPPD coding sequence in the plant genome.

In the embodiments herein, "base editing fusion protein" and "base editor" are used interchangeably and refer to those proteins that can mediate the substitution of one or more nucleotides of a target sequence in the genome in a sequence-specific manner.

As used herein, the term "CRISPR effector protein" generally refers to a nuclease (CRISPR nuclease) or a functional variant thereof found in a naturally occurring CRISPR system. The term covers any effector protein based on the CRISPR system that can achieve sequence-specific targeting in cells.

As used herein, a "functional variant" in terms of CRISPR nuclease means that it retains at least the guide RNA-mediated sequence-specific targeting ability. Preferably, the functional variant is a nuclease inactivated variant, that is, it lacks double-stranded nucleic acid cleavage activity. However, CRISPR nucleases lacking double-stranded nucleic acid cleavage activity also encompass nickases, which form a nick in the double-stranded nucleic acid molecule, but do not completely cut the double-stranded nucleic acid. In some preferred embodiments of the present invention, the CRISPR effector protein of the present invention has nickase activity. In some embodiments, the functional variant recognizes a different PAM (protospacer adjacent motif) sequence relative to the wild-type nuclease.

The "CRISPR effector protein" can be derived from Cas9 nuclease, including Cas9 nuclease or functional variants thereof. The Cas9 nuclease may be a Cas9 nuclease from different species, such as spCas9 from S. pyogenes or SaCas9 derived from S. aureus. "Cas9 nuclease" and "Cas9" are used interchangeably herein and refer to RNA guided nuclease comprising Cas9 protein or fragments thereof (for example, a protein containing the active DNA cleavage domain of Cas9 and/or the gRNA binding domain of Cas9). Cas9 is a component of the CRISPR/Cas (clustered regularly spaced short palindrome repeats and related systems) genome editing system, which can target and cleave the DNA target sequence under the guidance of the guide RNA to form a DNA double-strand break (DSB). An exemplary amino acid sequence of wild-type spCas9 is shown in SEQ ID NO: 17.

The "CRISPR effector protein" can also be derived from Cpfl nuclease, including Cpfl nuclease or functional variants thereof. The Cpfl nuclease may be Cpfl nuclease from different species, such as Cpfl nuclease from Francisella novicida U112, Acidaminococcus sp. BV3L6 and Lachnospiraceae bacterium ND2006.

Available "CRISPR effector proteins" can also be derived from Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Csn2, Cas4, C2c1, C2c3 or C2c2 nucleases, for example, include these nucleases or functional variants thereof.

In some embodiments, the CRISPR effector protein is a nuclease-inactivated Cas9. The DNA cleavage domain of Cas9 nuclease is known to contain two subdomains: HNH nuclease subdomain and RuvC subdomain. The HNH subdomain cleaves the strand complementary to the gRNA, while the RuvC subdomain cleaves the non-complementary strand. Mutations in these subdomains can inactivate the nuclease activity of Cas9, forming "nuclease-inactivated Cas9". The nuclease-inactivated Cas9 still retains the DNA binding ability guided by gRNA.

The nuclease-inactivated Cas9 of the present invention can be derived from Cas9 of different species, for example, derived from S. pyogenes Cas9 (SpCas9), or derived from Staphylococcus aureus (S. aureus) Cas9 (SaCas9). Simultaneously mutating the HNH nuclease subdomain and RuvC subdomain of Cas9 (for example, including the mutations D10A and H840A) inactivates the nuclease of Cas9 and becomes the nuclease death Cas9 (dCas9). Mutation and inactivation of one of the subdomains can allow Cas9 to have nickase activity, that is, to obtain a Cas9 nickase (nCas9), for example, nCas9 with only mutation D10A.

Accordingly, in some embodiments of the invention, the nuclease-inactivated Cas9 variants of the invention comprise the amino acid substitutions D10A and/or H840A relative to wild-type Cas9, wherein the amino acid numbering refers to SEQ ID NO:17. In some preferred embodiments, the nuclease-inactivated Cas9 comprises the amino acid substitution D10A relative to wild-type Cas9, wherein the amino acid numbering refers to SEQ ID NO: 17. In some embodiments, the nuclease-inactivated Cas9 comprises the amino acid sequence set forth in SEQ ID NO: 18 (nCas9 (D10A)).

When Cas9 nuclease is used for gene editing, it usually requires the target sequence to have a 5'-NGG-3' PAM (protospacer adjacent motif) sequence at the 3'end. However, CRISPR effector proteins that recognize different PAM sequences can also be used, such as Cas9 nuclease functional variants with different PAM sequences.

In some embodiments, the CRISPR effector protein is a Cas9 variant that recognizes the PAM sequence 5'-NG-3'. Cas9 variants that recognize the PAM sequence 5'-NG-3' are also referred to herein as Cas9-NG. In some embodiments, Cas9-NG comprises the following amino acid substitutions R1335V, L1111R, D1135V, G1218R, E1219F, A1322R, T1337R relative to wild-type Cas9, wherein amino acid numbering refers to SEQ ID NO:17. In some embodiments, the CRISPR effector protein is a nuclease inactivated Cas9 variant that recognizes the PAM sequence 5'-NG-3'. In some embodiments, the nuclease-inactivated Cas9 variant that recognizes the PAM sequence 5'-NG-3' comprises the following amino acid substitutions D10A, R1335V, L1111R, D1135V, G1218R, E1219F, A1322R, T1337R relative to wild-type Cas9, wherein the amino acid numbering refers to SEQ ID NO:17. In some embodiments, the nuclease-inactivated Cas9 variant that recognizes the PAM sequence 5'-NG-3' comprises the amino acid sequence set forth in SEQ ID NO: 19 (nCas9-NG(D10A)).

In some embodiments, the CRISPR effector protein is a Cas9 variant that recognizes the PAM sequence 5'-NGA-3', ie, VQR-Cas9. In some embodiments, VQR-Cas9 comprises the following amino acid substitutions relative to wild-type Cas9: D1135V, R1335Q, and T1337R, wherein the amino acid numbering refers to SEQ ID NO:17. In some embodiments, the CRISPR effector protein is a nuclease-inactivated Cas9 variant that recognizes the PAM sequence 5'-NGA-3'. In some embodiments, the nuclease-inactivated Cas9 variant that recognizes the PAM sequence 5'-NGA-3' comprises the following amino acid substitutions D10A, D1135V, R1335Q, and T1337R relative to wild-type Cas9, wherein amino acid numbering refers to SEQ ID NO :17. In some embodiments, the nuclease-inactivated Cas9 variant that recognizes the PAM sequence 5'-NG-3' comprises the amino acid sequence set forth in SEQ ID NO: 20 (nCas9-VQR(D10A)).

The deaminase domain described herein may be a cytosine deaminase domain or an adenine deaminase domain.

As used herein, "cytosine deamination domain" refers to a domain that can accept single-stranded DNA as a substrate and catalyze the deamination of cytidine or deoxycytidine into uracil or deoxyuracil, respectively.

Examples of cytosine deaminase that can be used in the present invention include, but are not limited to, for example, APOBEC1 deaminase, activation-induced cytidine deaminase (AID), APOBEC3G, CDA1, human APOBEC3A deaminase, or their functional variants. In some embodiments, the cytosine deaminase is human APOBEC3A deaminase or a functional variant thereof. In some embodiments, the human APOBEC3A deaminase comprises the amino acid sequence set forth in SEQ ID NO:21. In some embodiments, the cytosine deaminase is PmCDA1. In some embodiments PmCDA1 comprises the amino acid sequence set forth in SEQ ID NO:22.

As used herein, "adenine deamination domain" refers to a domain that can accept single-stranded DNA as a substrate and catalyze the formation of inosine (I) from adenosine or deoxyadenosine (A). In some embodiments, the adenine deaminase is a variant of E. coli tRNA adenine deaminase TadA (ecTadA), for example, comprising the amino acid sequence set forth in SEQ ID NO:23.

In some specific embodiments, the base editing fusion protein comprises the amino acid sequence set forth in SEQ ID NO: 24 or 25 or 89.

As used herein, "guide RNA" and "gRNA" are used interchangeably and refer to RNA that can form a complex with the CRISPR effector protein and can target the complex to the target sequence due to a certain sequence identity with the target sequence. The guide RNA targets the target sequence by base pairing with the complementary strand of the target sequence. For example, the gRNA used by Cas9 nuclease or its functional variants is usually composed of crRNA and tracrRNA molecules that are partially complementary to form a complex, wherein the crRNA contains a guide sequence (also called a seed sequence) having sufficient identity with the target sequence so as to hybridize with the complementary strand of the target sequence and guide the CRISPR complex (Cas9+crRNA+tracrRNA) to specifically bind to the target sequence. However, it is known in the art that single guide RNA (sgRNA) can be designed, which includes both the characteristics of crRNA and tracrRNA. The gRNA used by the Cpfl nuclease or its functional variants is usually composed only of mature crRNA molecules, which can also be called sgRNA. Designing a suitable gRNA based on the CRISPR nuclease used and the target sequence to be edited is within the abilities of those skilled in the art.

In some embodiments, the at least one gRNA of the present invention comprises a target sequence in the endogenous ALS coding region, and the amino acid sequence encoded by the target sequence comprises the amino acid(s) of the endogenous ALS at one or more positions selected from the group consisting of position 99, 98, 101 and 102, the ALS amino acid positions referring to SEQ ID NO:1. In some specific embodiments, the at least one gRNA of the present invention comprises the nucleotide sequence set forth in one of SEQ ID NOs: 26-27 or the complement thereof.

In some embodiments, the at least one gRNA of the present invention comprises a target sequence in the endogenous PPO coding region, and the amino acid sequence encoded by the target sequence comprises the amino acid(s) of the endogenous PPO at one or more positions selected from the group consisting of position 127 and 129, the PPO amino acid positions referring to SEQ ID NO:9. In some specific embodiments, the at least one gRNA of the present invention comprises the nucleotide sequence set forth in one of SEQ ID NOs: 28-30 or the complement thereof.

In some embodiments, the at least one gRNA of the present invention comprises a target sequence in the endogenous ACCase coding region, and the amino acid sequence encoded by the target sequence comprises the amino acid(s) of the endogenous ACCase at one or more positions selected from the group consisting of position 1866, 1884, 1927 or 2181, the ACCase amino acid positions referring to SEQ ID NO:31. In some specific embodiments, the at least one gRNA of the present invention comprises the nucleotide sequence set forth in one of SEQ ID NOs: 41-44 or the complement thereof.

In some embodiments, the at least one gRNA of the present invention comprises a target sequence in the endogenous HPPD coding region, and the amino acid sequence encoded by the target sequence comprises the amino acid(s) of the endogenous HPPD at one or more positions selected from the group consisting of position 365, 379, 353, 354, 346, 347, 369 or 370, the HPPD amino acid positions referring to SEQ ID NO:45. In some specific embodiments, the at least one gRNA of the present invention comprises the nucleotide sequence set forth in one of SEQ ID NOs: 52-88 or the complement thereof.

In the method of the present invention, the base editing system can be introduced into plants by various methods well known to those skilled in the art. Methods that can be used to introduce the base editing system of the present invention into plants include, but are not limited to gene bombardment, PEG-mediated transformation of protoplasts, Agrobacterium-mediated transformation, plant virus-mediated transformation, pollen tube approach, and ovary injection approach.

In the method of the present invention, the modification of the target sequence can be achieved by only introducing or producing the base editing fusion protein and the guide RNA in the plant cell, and the modification can be stably inherited, without any need to stably transform the gene editing system into plants. This avoids the potential off-target effect of the stable gene editing system and also avoids the integration of the exogenous nucleotide sequence in the plant genome, thereby providing greater biosafety.

In some preferred embodiments, the introduction is carried out in the absence of selection pressure to avoid integration of the exogenous nucleotide sequence into the plant genome.

In some embodiments, the introduction comprises transforming the base editing system of the present invention into an isolated plant cell or tissue and then regenerating the transformed plant cell or tissue into an intact plant. Preferably, the regeneration is carried out in the absence of selection pressure, i.e., no selection agent for the selection gene on the expression vector is used during tissue culture. Avoiding the use of a selection agent can increase the regeneration efficiency of the plant, obtaining a modified plant free of exogenous nucleotide sequences.

In other embodiments, the base editing system of the present invention can be transformed into specific parts of an intact plant, such as leaves, shoot tips, pollen tubes, young ears or hypocotyls. This is particularly suitable for the transformation of plants that are difficult to regenerate in tissue culture.

In some embodiments of the present invention, proteins expressed in vitro and/or RNA molecules transcribed in vitro are directly transformed into the plant. The protein and/or RNA molecule can achieve base editing in plant cells and then be degraded by the cell, avoiding the integration of foreign nucleotide sequences in the plant genome.

In another aspect, the present invention also provides a herbicide-resistant plant produced by the base editing method of the present invention. The present invention also encompasses the progeny of the herbicide-resistant plant.

In some embodiments of the invention, the herbicide-resistant plant is transgen-free.

The plants described in various aspects of the invention may be plants that are susceptible to ALS inhibitors and/or PPO inhibitors and/or ACCase inhibitors and/or HPPD inhibitors, including monocotyledonous or dicotyledonous plants. Preferably, the plant is a crop plant, such as a monocotyledonous crop plant. Examples of suitable plants include, but are not limited to, rice, wheat, barley, sorghum, corn, and the like. In some preferred embodiments, the plant is wheat. In some preferred embodiments, the plant is rice.

In some embodiments, the plant is wheat, and the endogenous ALS and/or endogenous PPO are encoded by the wheat A genome, B genome, and/or D genome.

### 9. Methods of generating herbicide-resistant plants by physical or chemical mutagenesis

In another aspect, the present invention also provides a method for producing herbicide-resistant plants, comprising physical or chemical mutagenesis of a population of the plant, and screening a plant comprising
(i) amino acid mutation in the endogenous ALS at one or more positions selected from the group consisting of positions 99, 98, 101 and 102, said ALS amino acid position referring to SEQ ID NO: 1, and/or
(ii) amino acid mutation in the endogenous PPO at one or more positions selected from the group consisting of positions 127 and 129, the PPO amino acid positions referring to SEQ ID NO: 9, and/or
(iii) amino acid mutation in the endogenous ACCase at one or more positions selected from the group consisting of positions 1866, 1884, 1927 or 2181, said ACCase amino acid positionsreferring to SEQ ID NO: 31, and/or
(iv) amino acid mutation in the endogenous HPPD at one or more positions selected from the group consisting of positions 365, 379, 353, 354, 346, 347, 369 or 370, the HPPD amino acid position referring to SEQ ID NO:45.

In some embodiments, the method comprises screening a plant in which the alanine (A) at position 99 and/or the glycine (G) at position 98 and/or the methionine (M) at position 101 and/or glutamic acid (E) at position 102 in the endogenous ALS is substituted, with the ALS amino acid position referring to SEQ ID NO:1.

In some embodiments, the method comprises screening a plant in which the alanine (A) at position 99 is substituted with a threonine (T) and/or the glycine (G) at position 98 is substituted with asparagine (N) and/or the methionine (M) at position 101 is substituted with isoleucine (I) and/or glutamic acid (E) at position 102 in the endogenous ALS is substituted by lysine (K), with the ALS amino acid position referring to SEQ ID NO:1.

In some embodiments, the method comprises screening a plant in which the endogenous ALS comprises one or more amino acid substitutions selected from the group consisting of A99T, G98N, M101I, E102K, the ALS amino acid positions referring to SEQ ID NO:1.

In some embodiments, the method comprises screening a plant in which the arginine (R) at position 127 and/or the threonine (T) at position 129 of the endogenous PPO is substituted, the PPO amino acid position referring to SEQ ID NO:9.

In some embodiments, the method comprises screening a plant in which that the arginine (R) at position 127 is substituted with a cysteine (C) or a glycine (G) and/or the threonine (T) at position 129 of the endogenous PPO is substituted with isoleucine (I), the PPO amino acid position referring to SEQ ID NO:9.

In some embodiments, the method comprises screening a plant in which the endogenous PPO comprises one or more amino acid substitutions selected from the group consisting of R127C, R127G, T129I, the PPO amino acid positions referring to SEQ ID NO:9.

In some embodiments, the method comprises screening a plant in which in the endogenous ACCase, the serine (S) at position 1866 is substituted, and/or the alanine (A) at position 1884 is substituted, and/or the proline (P) at position 1927 is substituted, and/or the proline (P) at position 2181 is substituted, the ACCase amino acid position referring to SEQ ID NO:31.

In some embodiments, the method comprises screening a plant in which in the endogenous ACCase, the serine (S) at position 1866 is substituted by phenylalanine (F), and/or the alanine (A) at position 1884 is substituted with proline (P), and/or the proline (P) at position 1927 is substituted with phenylalanine (F), and/or the proline (P) at position 2181 is substituted with leucine (L), the ACCase amino acid position referring to SEQ ID NO:31.

In some embodiments, the method comprises screening a plant in which the endogenous ACCase comprises one or more amino acid substitutions selected from the group consisting of S1866F, A1884P, P1927, P2181L, the ACCase amino acid positions referring to SEQ ID NO:31.

In some embodiments, the method comprises screening a plant in which the leucine (L) at position 365 and/or the threonine (T) at position 379, and/or the glutamic acid (E) at position 353, and/or the glutamic acid (E) at position 354, and/or the arginine (R) at position 346, and/or the alanine (A) at position 347, and/or the aspartic acid (D) at position 369, and/or the aspartic acid (D) at position 370 of the endogenous HPPD is substituted, with the HPPD amino acid positions referring to SEQ ID NO:45.

In some embodiments, the method comprises screening a plant in which the leucine (L) at position 365 is substituted by Phenylalanine (F) and/or the threonine (T) at position 379 is substituted by Isoleucine (I), and/or the glutamic acid (E) at position 353 is substituted by lysine (K), and/or the glutamic acid (E) at position 354 is substituted by lysine (K), and/or the arginine (R) at position 346 is substituted by cysteine (C), and/or the alanine (A) at position 347 is substituted by Valine (V), and/or the aspartic acid (D) at position 369 is substituted by Asparagine (N), and/or the aspartic acid (D) at position 370 of the endogenous HPPD is substituted by Asparagine (N), with the HPPD amino acid positions referring to SEQ ID NO:45.

In some embodiments, the method comprises screening a plant in which the endogenous HPPD comprises one or more amino acid substitutions selected from the group consisting of L365F, T379I, E353K, E354K, E353K/E354K, R346C, A347V, R346C/A347V, D369N, D370N, D369N/D370N, the HPPD amino acid positions referring to SEQ ID NO:45.

∘ In some embodiments, the physical mutagenesis can be achieved by radioactively irradiating the plant population. In some embodiments, the chemical mutagenesis can be achieved by treating the plant population (for example, treating plant seeds) with ethyl methanesulfonate (EMS).

In some embodiments, the screening can be achieved by sequencing the coding sequence of the endogenous ALS and/or the endogenous PPO and/or the endogenous ACCase and/or the endogenous HPPD.

In another aspect, the present invention also provides a herbicide-resistant plant produced by the physical or chemical mutagenesis method of the present invention. The present invention also encompasses the progeny of the herbicide-resistant plant.

The plants described in various aspects of the invention may be plants that are susceptible to ALS inhibitors and/or PPO inhibitors and/or ACCase inhibitors and/or HPPD inhibitors, including monocotyledonous or dicotyledonous plants. Preferably, the plant is a crop plant, such as a monocotyledonous crop plant. Examples of suitable plants include, but are not limited to, rice, wheat, barley, sorghum, corn, and the like. In some preferred embodiments, the plant is wheat. In some preferred embodiments, the plant is rice.

### 10. Plant breeding methods

In another aspect, the present invention provides a plant breeding method, comprising crossing a first plant having herbicide resistance obtained by the above method of the present invention with a second plant not containing the herbicide resistance, thereby the herbicide resistance is introduced into the second plant.

The plants described in various aspects of the invention may be plants that are susceptible to ALS inhibitors and/or PPO inhibitors and/or ACCase inhibitors and/or HPPD inhibitors, including monocotyledonous or dicotyledonous plants. Preferably, the plant is a crop plant, such as a monocotyledonous crop plant. Examples of suitable plants include, but are not limited to, rice, wheat, barley, sorghum, corn, and the like. In some preferred embodiments, the plant is wheat. In some preferred embodiments, the plant is rice.

### Examples

### Example 1. Generation and identification of wheat ALS-resistant mutants

### 1. Single base editing tools as used

The PmCDA1-VQR-PBE single base editing system fused to PmCDA1 cytosine deaminase was used. The sequence of PmCDA1 cytosine deaminase is from Nishida, K., et al. (2016). Targeted nucleoffide editing using hybrid prokaryoffic and vertebrate adaptive immune systems. Science 353, aaf8729. VQR-Cas9 is a variant of SpCas9 that recognizes NGA-type PAM sequences. PmCDA1 cytosine deaminase, VQR-Cas9 (D10A) and UGI moieties were codon-optimized in cereals and synthesized commercially (GENEWIZ, Suzhou, China). Various structures were assembled into the pJIT163 backbone by the Gibson assembly method (Mut Express MultiS Fast Mutagenesis Kit, Vazyme, Nanjing, China). PCR in this work was performed using TransStart FastPfu DNA polymerase (TransGen Biotech).

### 2. Construction of multi-target sgRNA and crRNA expression vectors

Target site primers with homologous sequences were designed and synthesized:
T1(W16-B)-F: GGGACGCGCCGCCAGGGTAGGGTTTTAGAGCTAGAAATAGC
T1(W16-B)-BSF: ATATTGGTCTCTCTTGGGACGCGCCGCCAGGGTAGGGTT
T2(W1)-R: CATCATGCGGCGGGGGACCTGCGCTTCTTGGTGCCGCGCC
T2(W1)-F: GCAGGTCCCCCGCCGCATGATGTTTTAGAGCTAGAAATAGC
T3(W16-D)-R: CCCTACCCCGGCGGCGCCTCCCGCCACGGATCATCTGCACAAC
T3(W16-D)-B SR: TTATAGGTCTCTAAACCCTACCCCGGCGGCGCCTCCCGC

According to the method of Xing, H. L. et al. (A CRISPR/Cas9 toolkit for multiplex genome editing in plants. BMC Plant Biol. 14, 327 (2014)), sgRNA expression vectors for two target sequences (SEQ ID NOs: 26 and 27, located in the B genome ALS coding sequence and the D genome ALS coding sequence, respectively) were constructed.

### 3. Gene bombardment transformation

The variety of wheat (Triticum aestivum L.) used was Konon 199, a winter wheat. The PmCDA1-VQR-PBE plasmid and the sgRNA plasmid were mixed at a molar ratio of 1:1 using the gene gun-mediated transformation method to bombard immature wheat embryos. For the specific process, refer to Rasco-Gaunt (2001) Procedures allowing the transformation of a range of European elite wheat (Triticum aestivum L.) varieties via particle bombardment. J. Exp. Bot. 52, 865-874.

### 4. Establishment of herbicide screening system

The existing imazapic-resistant wheat plants were used as positive control, and wild-type wheat was used as negative control. Different concentrations of imazapic were added to the wheat rooting medium, and an appropriate screening concentration was found, that is, 1/15 of the concentration of imazapic used in the field. At this concentration, the rooting of wild-type wheat was significantly inhibited, while the overexpression-positive wheat could grow roots normally.

### 5. Genotyping

The screened positive plants were sampled, and a small amount of plant genomic DNA was extracted using the Tiangen Express Plant Genome Extraction Kit (Tiangen DP321), and the specific genotype was determined after Sanger sequencing. The results are shown in Table 1 below.

**Table 1. Types of amino acid mutations in TaALS-resistant mutant wheat (Genome A-A99 corresponds to Genome B-A98 and Genome D-A96)**

| Mutant No. | A-genome | B-genome | D-genome |
|---|---|---|---|
| 1 | WT | [A98T] | [A96T] |
| 2 | [A99T]/WT | [A98T] | [A96T] |
| 3 | [A99T]/WT | [A98T] | [A96T] |
| 4 | [A99T]/WT | [E101K,M100I,A98T,G97N]/[A98T] | [A96T] |
| 5 | [A99T]/WT | [E101K,M100I,A98T,G97N]/[A98T] | [A96T] |
| 6 | [A99T]/WT | [E101K,M100I,A98T,G97N]/[A98T] | [A96T] |
| 7 | [A99T]/WT | [E101K,M100I,A98T,G97N]/[A98T] | [A96T] |
| 8 | [A99T] | [A98T] | [A96T] |
| 9 | [A99T] | [E101K,M100I,A98T,G97N] | [A96T] |
| 10 | [A99T] | [E101K,M100I,A98T,G97N] | [A96T] |
| 11 | [A99T] | [E101K,M100I,A98T,G97N]/[A98T] | [A96T] |
| 12 | [A99T] | [E101K,M100I,A98T,G97N]/[A98T,G97N] | [A96T] |

### Example 2. Generation and identification of wheat PPO-resistant mutants

### 1. Single base editing tools as used

A3A-PBE system with hAPOBEC3A cytosine deaminase fusion was used (5. Zong, Y., et al. (2018). "Efficient C-to-T base editing in plants using a fusion of nCas9 and human APOBEC3A." Nat. Biotechnol. 36(10): 950).

### 2. Construction of multi-target sgRNA expression vectors

Target site primers with homologous sequences were designed and synthesized:

| | |
|---|---|
| T1(R98-AD)-F | GAAGCGTTATACTGTTAAGGAGTTTTAGAGCTAGAAATAGC |
| T1(R98-AD)-BsF | ATATTGGTCTCTCTTGAAGCGTTATACTGTTAAGGAGTT |
| T2(W1)-R | CATCATGCGGCGGGGGACCTGCGCTTCTTGGTGCCGCGCC |
| T2(W1)-F | GCAGGTCCCCCGCCGCATGATGTTTTAGAGCTAGAAATAGC |
| T3(R98-B)-R | CTTCTTAACAGTATAACGCTTCGCCACGGATCATCTGCACAAC |
| T3(R98-B)-BsR | TTATAGGTCTCTAAACTTCTTAACAGTATAACGCTTCGC |

According to the method of Xing, H. L. et al. (A CRISPR/Cas9 toolkit for multiplex genome editing in plants. BMC Plant Biol. 14, 327 (2014)), sgRNA expression vectors for three target sequences (SEQ ID NOs: 28-30, located in the A genome PPO coding sequence, the B genome PPO coding sequence and the D genome PPO coding sequence, respectively) were constructed.

### 3. Gene bombardment transformation

The variety of wheat (Triticum aestivum L.) used was Konon 199, a winter wheat. The A3A-PBE plasmid and the sgRNA plasmid were mixed at a molar ratio of 1:1 using the gene gun-mediated transformation method to bombard immature wheat embryos. For the specific process, refer to Rasco-Gaunt (2001) Procedures allowing the transformation of a range of European elite wheat (Triticum aestivum L.) varieties via particle bombardment. J. Exp. Bot. 52, 865-874.

### 4. Establishment of herbicide screening system

The existing wheat plants overexpressin PPO protein were used as positive control, and wild-type wheat was used as negative control. Different concentrations of lactoferpodium were added to the wheat rooting medium, and an appropriate screening concentration was found, that is, 1/6 of the concentration of lactoferpodiumused in the field. At this concentration, the rooting of wild-type wheat was significantly inhibited, while the overexpression-positive wheat could grow roots normally.

### 5. Genotyping

The screened positive plants were sampled, and a small amount of plant genomic DNA was extracted using the Tiangen Express Plant Genome Extraction Kit (Tiangen DP321), and the specific genotype was determined after Sanger sequencing. The results are shown in Table 2 below.

**Table 2. Types of amino acid mutations in PPO-resistant mutant wheat (Genome A-R127 corresponds to Genome B-R129 and Genome D-R133)**

| Mutant No. | A-R127 | B-R129 | D-R133 |
|---|---|---|---|
| 1 | WT | WT | [R133C]/WT |
| 2 | WT | WT | [R133C]/WT |
| 3 | [R127C] | WT | [R133C] |
| 4 | [R127C] | WT | [R133C] |
| 5 | [R127C] | WT | [R133C] |
| 6 | [R127C] | WT | [R133C] |
| 7 | [R127C] | [R129C]/WT | [R133C] |
| 8 | [R127C] | [R129C]/WT | [R133C] |
| 9 | [R127C] | [R129C] | [R133C] |
| 10 | [R127C] | [R129C] | [R133C] |
| 11 | [R127C] | [R129C]/WT | [R133C,T135I]/WT |
| 12 | WT/[R127G] | WT | [R133C]/WT |

### Example 3 Generation and identification of rice ACCase-resistant mutants

### 3.1 Construction of A3A-PBE-NG vector

Using the pH-A3A-PBE vector (Q. Shan et al., Targeted genome modification of crop plants using a CRISPR-Cas system. Nature Biotechnology 31, 686 (2013)) as the basic backbone, the nCas9 part was replaced by the nCas9-NG containing D10A/R1335V/L1111R/D1135V/G1218R/E1219F/A1322R/T1337R mutations (Y. Zong et al., Efficient C-to-T base editing in plants using a fusion of nCas9 and human APOBEC3A. Nat Biotechnol, (2018)) by Gibson assembly method to obtain a base editing vector A3A-PBE-NG capable of recognizing the NG PAM sequence. The nCas9-NG sequence was synthesized by GENEWIZ (GENEWIZ, Suzhou, China).

### 3.2 Construction of base editing vector targeting rice OsACC gene

Four target sequences targeting the carboxyltransferase (CT) domain of rice OsACC were selected (see Table 3), and the base editing vector for different sites in Table 3 were constructed according to the sgRNA vector construction method (same as above) reported by Yuan Zong.

**Table 3**

| **Target site** | **target site sequence** |
|---|---|
| OsACC-S1866 | ATTGATTCTGTTGTGGGCAAGG |
| OsACC-A1884 | CATGGAAGTGCTGCTATTGCCA |
| OsACC-P1927 | ACCAGCCTATTATTCTTACAGG |
| OsACC-P2181 | AAGATAAACCCAGACCGCATTG |

| | |
|---|---|
| Note: The underline is the PAM sequence | |

### 3.3 Agrobacterium-transformation of rice

The A3A-PBE-NG vector containing the sgRNA coding sequence for the target site was transformed into the Agrobacterium AGL1 strain by electroporation. Agrobacterium-mediated transformation, tissue culture and regeneration of rice cultivar Zhonghua 11 was performed according to Shan et al. Hygromycin selection (50 µg/ml) was used throughout the tissue culture process.

### 3.4 Screening and identification of resistant plants

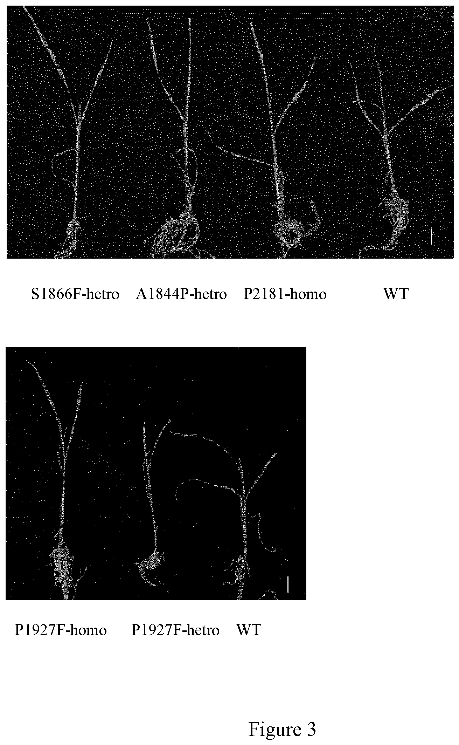

According to the growth state of the regenerated plants, the T0 generation plants regenerated on the medium were placed in water for 5 to 30 days, and then the plant stems and leaves were directly sprayed with Haloxyfop-P, and the spray concentration range is between 0.1 and 2 times of the recommended concentration of commercial preparations. Four weeks after spraying, a total of 5 surviving mutants, ie, herbicide-resistant mutants, were obtained (Fig. 3).

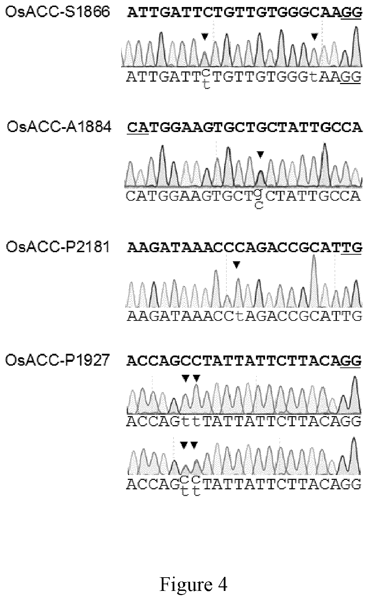

The DNA of the surviving mutants was extracted, the OsACC region containing the target sequence was amplified by PCR, and the mutation type of the resistant mutants was determined by Sanger sequencing. The sequencing results of the resistant mutants are shown in Figure 4, in which the C to T mutation generated at position 8 of the OsACC-S1866 target sequence (defining NG PAM as positions 21 and 22 of the sgRNA target site) caused the mutation of S1866F. Although the 18th position of the OsACC-S1866 target sequence also produced a C to T mutation but did not cause an amino acid change; G to C mutation at the 10th position of the OsACC-A1884 target sequence caused the amino acid change of A1884P; The C-to-T mutation at the 11th position of the OsACC-P2181 target sequence caused the amino acid change of P2181L; the C-to-T mutation at the 6th and 7th position of the OsACC-P1927 target sequence caused the amino acid change of P1927F.

### Example 4 Generation and identification of rice HPPD-resistant mutants

### 4.1 Construction of base editing vector for rice HPPD

The A3A-PBE with fused hAPOBEC3A cytosine deaminase was constructed according to literatures (Zong, Y., et al. (2018). "Efficient C-to-T base editing in plants using a fusion of nCas9 and human APOBEC3A." Nat. Biotechnol. 36(10): 950), and the A3A-NG-PBE was obtained by replacing the wild-type SpCas9 with the SpCas9-NG variant. PCR during vector construction was performed using TransStart FastPfu DNA polymerase (TransGen Biotech).

The target sequence of the gRNA for rice HPPD was designed according to the coding sequence of rice HPPD (SEQ ID NO: 51), as shown in Table 4 below.

**Table 4. Rice HPPD target sequence, divided into: NG PAM target sequences (including NGG), that is, the target sequences are located on the coding strand; CN PAM target sequence (including CCN), that is, the target sequences are located on the antisense strand. (The underlined is the PAM sequence)**

| **NG(NGG)** | **CN(CCN)** |
|---|---|
| GTGCGGCGGCGCGCCGGGGACG | CCCTTGAAGCCAAGCAAGCCCC |
| GCGCGCCGGGGACGTGCTCTCGG | CAAGTCCATTGAGGAGTATGAG |
| GGACGTGCTCTCGGAGGAGCAG | CAACTTCTCGGAGCTGTTCAAG |
| CTCGGAGGAGCAGATCAACGAG | CTGCGGCGGGTTTGGGAAGGGC |
| TGCCAGGAGCTCGGGGTGCTCG | CAGGAGTACCAGAAGGGCGGCT |
| AGCTCGGGGTGCTCGTGGACAGG | CCAGAAGGGCGGCTGCGGCGGGT |
| TGACCAGGGGGTGTTGCTCCAG | CATGGAGAAGGATGAGAGTGGG |
| GTTGCTCCAGATCTTCACCAAG | CAAAGGATTGGGTGCATGGAGA |
| TCACCAAGCCAGTAGGAGACAGG | CTTGGAGATGATACAAAGGATT |
| GCCAACCTTTTTCTTGGAGATG | CCAACCTTTTTCTTGGAGATGAT |
| GATACAAAGGATTGGGTGCATGG | CAGCTATTTCACTCAGGCCAAC |
| AGTGGGCAGGAGTACCAGAAGGG | CTTCACCAAGCCAGTAGGAGAC |
| GAGTACCAGAAGGGCGGCTGCG | CAGGGATGACCAGGGGGTGTTG |
| CAACTTCTCGGAGCTGTTCAAG | CTCGTGGACAGGGATGACCAGG |
| CAAGTCCATTGAGGAGTATGAG | CTCGGGGTGCTCGTGGACAGGG |
| AAATCCCTTGAAGCCAAGCAAG | CGAGTGCCAGGAGCTCGGGGTG |
| GAAGCCAAGCAAGCCCCTACAG | CAGATCAACGAGTGCCAGGAGC |
| | CCGGGGACGTGCTCTCGGAGGAG |
| | CGGCGGCGCGCCGGGGACGTGC |
| | CGGCGTGCGGCGGCGCGCCGGG |

### 4.2 Agrobacterium-transformation of rice

The A3A-PBE or A3A-PBE-NG vector containing the sgRNA coding sequence for the target sites were transformed into the Agrobacterium AGL1 strain by electroporation. Agrobacterium-mediated transformation, tissue culture and regeneration of rice cultivar Nipponbare or Zhonghua 11 was performed according to Hiei et al. (Hiei, Y., and Komari, T. (2008). Agrobacterium-mediated transformation of rice using immature embryos or calli induced from mature seed. Nat. Protoc. 3, 824-834). Hygromycin selection was used throughout the tissue culture process.

### 4.3 Screening of resistant plants

The rice tissue culture seedlings overexpressing HPPD protein were used as the positive control, and the wild-type rice plants of the same stage and variety were used as the negative control. Each group of 8 plants was sprayed with different concentration gradients of herbicide: mesotrione or Topramezone. About two weeks later, under the appropriate screening concentration, the leaves of the wild-type rice tissue culture seedlings without resistance turned white and then withered slowly, while the leaves of the overexpressing positive rice tissue culture seedlings were normal and grew normally. The herbicide concentration, that is, 1/8 of the field concentration of mesotrione or Topramezone, is a suitable screening concentration. The rice plants obtained in Example 2 were treated at this screening concentration to obtain herbicide-resistant plants

### 4.4 Genotyping

The positive plants obtained in Example 3 were sampled, and a small amount of plant genomic DNA was extracted using the Tiangen Express Plant Genome Extraction Kit (Tiangen DP321), and the specific genotype was determined after Sanger sequencing. The results are shown in Table 5 below:

**Table 5. Resistant rice plants and amino acid mutation types thereof**

| Resistant mutant No. | amino acid mutation |
|---|---|
| M1 | L365F |
| M2 | T3791 |
| M3 | E353K & E354K |
| M4 | R346C & A347V |
| M5 | D369N & D370N |

### Sequences

>SEQ ID NO:1 TaALS-A amino acid sequence 
MAAATSPAVAFSGAAAAAAAMPKPARQPLPRHQPASRRALPARVVRCCAAPPAAATSAAPPATALR
PWGPSEPRKGADILVEALERCGIVDVFAYPGGASMEIHQALTRSPVITNHLFRHEQGEAFAASGYAR
ASGRVGVCVATSGPGATNLVSALADALLDSIPMVAITGQVPRRMIGTDAFQETPIVEVTRSITKHNY
LVLDVEDIPRVIQEAFFLASSGRPGPVLVDIPKDIQQQMAVPIWDTPMSLPGYIARLPKPPSTESLEQ
VLRLVGESRRPILYVGGGCAASGEELRRFVELTGIPVTTTLMGLGNFPSDDPLSLRMLGMHGTVYA
NYAVDKADLLLAFGVRFDDRVTGKIEAFASRSKIVHIDIDPAEIGKNKQPHVSICADVKLALQGLNA
LLNGSKAQQGLDFGPWHKELDQQKREFPLGFKTFGEAIPPQYAIQVLDELTKGEAIIATGVGQHQM
WAAQYYTYKRPRQWLSSSGLGAMGFGLPAAAGAAVANPGVTVVDIDGDGSFLMNIQELALIRIEN
LPVKVMILNNQHLGMVVQWEDRFYKANRAHTYLGNPENESEIYPDFVTIAKGFNVPAVRVTKKSE
VTAAIKKMLETPGPYLLDIIVPHQEHVLPMIPSGGAFKDMIMEGDGRTSY*
>SEQ ID NO:2 TaALS-B amino acid sequence 
MAAATSPAVAFSGAAAAAAAIPKPARQPLPRHQPASRRALPARIVRCCAASPAATSVAPPATALRPW
GPSEPRKGADILVEALERCGIVDVFAYPGGASMEIHQALTRSPVITNHLFRHEQGEAFAASGYARAS
GRVGVCVATSGPGATNLVSALADALLDSIPMVAITGQVPRRMIGTDAFQETPIVEVTRSITKHNYLV
LDVEDIPRVIQEAFFLASSGRPGPVLVDIPKDIQQQMAVPVWDTPMSLPGYIARLPKPPSTESLEQVL
RLVGESRRPILYVGGGCAASGEELRRFVELTGIPVTTTLMGLGNFPSDDPLSLRMLGMHGTVYANY
AVDKADLLLAFGVRFDDRVTGKIEAFASRSKIVHIDIDPAEIGKNKQPHVSICADVKLALQGLNALL
NGSKAQQGLDFGPWHKELDQQKREFPLGFKTFGEAIPPQYAIQVLDELTKGEAIIATGVGQHQMW
AAQYYTYKRPRQWLSSSGLGAMGFGLPAAAGAAVANPGVTVVDIDGDGSFLMNIQELALIRIENL
PVKVMILNNQHLGMVVQWEDRFYKANRAHTYLGNPENESEIYPDFVTIAKGFNVPAVRVTKKSEV
TAAIKKMLETPGPYLLDIIVPHQEHVLPMIPSGGAFKDMIMEGDGRTSY*
>SEQ ID NO:3 TaALS-D amino acid sequence 
MAAATSPAVAFSGATAAAMPKPARHPLPRHQPVSRRALPARVVRCCAASPAATSAAPPATALRPWG
PSEPRKGADILVEALERCGIVDVFAYPGGASMEIHQALTRSPVITNHLFRHEQGEAFAASGYARASG
RVGVCVATSGPGATNLVSALADALLDSIPMVAITGQVPRRMIGTDAFQETPIVEVTRSITKHNYLVL
DVEDIPRVIQEAFFLASSGRPGPVLVDIPKDIQQQMAVPVWDTPMSLPGYIARLPKPPSTESLEQVLR
LVGESRRPILYVGGGCAASGEELRRFVELTGIPVTTTLMGLGNFPSDDPLSLRMLGMHGTVYANYA
VDKADLLLAFGVRFDDRVTGKIEAFASRSKIVHIDIDPAEIGKNKQPHVSICADVKLALQGLNDLLN
GSKAQQGLDFGPWHKELDQQKREFPLGFKTFGEAIPPQYAIQVLDELTKGEAIIATGVGQHQMWA
AQYYTYKRPRQWLSSSGLGAMGFGLPAAAGAAVANPGVTVVDIDGDGSFLMNIQELALIRIENLP
VKVMILNNQHLGMVVQWEDRFYKANRAHTYLGNPENESEIYPDFVTIAKGFNVPAVRVTKKSEVT
AAIKKMLETPGPYLLDIIVPHQEHVLPMIPSGGAFKDMIMEGDGRTSY*
>SEQ ID NO:4 TaALS-A-A99T 
MAAATSPAVAFSGAAAAAAAMPKPARQPLPRHQPASRRALPARVVRCCAAPPAAATSAAPPATALR
PWGPSEPRKGADILVEALERCGIVDVFAYPGGTSMEIHQALTRSPVITNHLFRHEQGEAFAASGYAR
ASGRVGVCVATSGPGATNLVSALADALLDSIPMVAITGQVPRRMIGTDAFQETPIVEVTRSITKHNY
LVLDVEDIPRVIQEAFFLASSGRPGPVLVDIPKDIQQQMAVPIWDTPMSLPGYIARLPKPPSTESLEQ
VLRLVGESRRPILYVGGGCAASGEELRRFVELTGIPVTTTLMGLGNFPSDDPLSLRMLGMHGTVYA
NYAVDKADLLLAFGVRFDDRVTGKIEAFASRSKIVHIDIDPAEIGKNKQPHVSICADVKLALQGLNA
LLNGSKAQQGLDFGPWHKELDQQKREFPLGFKTFGEAIPPQYAIQVLDELTKGEAIIATGVGQHQM
WAAQYYTYKRPRQWLSSSGLGAMGFGLPAAAGAAVANPGVTVVDIDGDGSFLMNIQELALIRIEN
LPVKVMILNNQHLGMVVQWEDRFYKANRAHTYLGNPENESEIYPDFVTIAKGFNVPAVRVTKKSE
VTAAIKKMLETPGPYLLDIIVPHQEHVLPMIPSGGAFKDMIMEGDGRTSY*
>SEQ ID NO:5 TaALS-B-A98T 
MAAATSPAVAFSGAAAAAAAIPKPARQPLPRHQPASRRALPARIVRCCAASPAATSVAPPATALRPW
GPSEPRKGADILVEALERCGIVDVFAYPGGTSMEIHQALTRSPVITNHLFRHEQGEAFAASGYARAS
GRVGVCVATSGPGATNLVSALADALLDSIPMVAITGQVPRRMIGTDAFQETPIVEVTRSITKHNYLV
LDVEDIPRVIQEAFFLASSGRPGPVLVDIPKDIQQQMAVPVWDTPMSLPGYIARLPKPPSTESLEQVL
RLVGESRRPILYVGGGCAASGEELRRFVELTGIPVTTTLMGLGNFPSDDPLSLRMLGMHGTVYANY
AVDKADLLLAFGVRFDDRVTGKIEAFASRSKIVHIDIDPAEIGKNKQPHVSICADVKLALQGLNALL
NGSKAQQGLDFGPWHKELDQQKREFPLGFKTFGEAIPPQYAIQVLDELTKGEAIIATGVGQHQMW
AAQYYTYKRPRQWLSSSGLGAMGFGLPAAAGAAVANPGVTVVDIDGDGSFLMNIQELALIRIENL
PVKVMILNNQHLGMVVQWEDRFYKANRAHTYLGNPENESEIYPDFVTIAKGFNVPAVRVTKKSEV
TAAIKKMLETPGPYLLDIIVPHQEHVLPMIPSGGAFKDMIMEGDGRTSY*
>SEQ ID NO:6 TaALS-B-A98T/G97N 
MAAATSPAVAFSGAAAAAAAIPKPARQPLPRHQPASRRALPARIVRCCAASPAATSVAPPATALRPW
GPSEPRKGADILVEALERCGIVDVFAYPGNTSMEIHQALTRSPVITNHLFRHEQGEAFAASGYARAS
GRVGVCVATSGPGATNLVSALADALLDSIPMVAITGQVPRRMIGTDAFQETPIVEVTRSITKHNYLV
LDVEDIPRVIQEAFFLASSGRPGPVLVDIPKDIQQQMAVPVWDTPMSLPGYIARLPKPPSTESLEQVL
RLVGESRRPILYVGGGCAASGEELRRFVELTGIPVTTTLMGLGNFPSDDPLSLRMLGMHGTVVYANY
AVDKADLLLAFGVRFDDRVTGKIEAFASRSKIVHIDIDPAEIGKNKQPHVSICADVKLALQGLNALL
NGSKAQQGLDFGPWHKELDQQKREFPLGFKTFGEAIPPQYAIQVLDELTKGEAIIATGVGQHQMW
AAQYYTYKRPRQWLSSSGLGAMGFGLPAAAGAAVANPGVTVVDIDGDGSFLMNIQELALIRIENL
PVKVMILNNQHLGMVVQWEDRFYKANRAHTYLGNPENESEIYPDFVTIAKGFNVPAVRVTKKSEV
TAAIKKMLETPGPYLLDIIVPHQEHVLPMIPSGGAFKDMIMEGDGRTSY*
>SEQ ID NO:7 TaALS-B-E101K/M100I/A98T/G97N 
MAAATSPAVAFSGAAAAAAAIPKPARQPLPRHQPASRRALPARIVRCCAASPAATSVAPPATALRPW
GPSEPRKGADILVEALERCGIVDVFAYPGNTSIKIHQALTRSPVITNHLFRHEQGEAFAASGYARASG
RVGVCVATSGPGATNLVSALADALLDSIPMVAITGQVPRRMIGTDAFQETPIVEVTRSITKHNYLVL
DVEDIPRVIQEAFFLASSGRPGPVLVDIPKDIQQQMAVPVWDTPMSLPGYIARLPKPPSTESLEQVLR
LVGESRRPILYVGGGCAASGEELRRFVELTGIPVTTTLMGLGNFPSDDPLSLRMLGMHGTVYANYA
VDKADLLLAFGVRFDDRVTGKIEAFASRSKIVHIDIDPAEIGKNKQPHVSICADVKLALQGLNALLN
GSKAQQGLDFGPWHKELDQQKREFPLGFKTFGEAIPPQYAIQVLDELTKGEAIIATGVGQHQMWA
AQYYTYKRPRQWLSSSGLGAMGFGLPAAAGAAVANPGVTVVDIDGDGSFLMNIQELALIRIENLP
VKVMILNNQHLGMVVQWEDRFYKANRAHTYLGNPENESEIYPDFVTIAKGFNVPAVRVTKKSEVT
AAIKKMLETPGPYLLDIIVPHQEHVLPMIPSGGAFKDMIMEGDGRTSY*
>SEQ ID NO:8 TaALS-D-A96T 
MAAATSPAVAFSGATAAAMPKPARHPLPRHQPVSRRALPARVVRCCAASPAATSAAPPATALRPWG
PSEPRKGADILVEALERCGIVDVFAYPGGTSMEIHQALTRSPVITNHLFRHEQGEAFAASGYARASG
RVGVCVATSGPGATNLVSALADALLDSIPMVAITGQVPRRMIGTDAFQETPIVEVTRSITKHNYLVL
DVEDIPRVIQEAFFLASSGRPGPVLVDIPKDIQQQMAVPVWDTPMSLPGYIARLPKPPSTESLEQVLR
LVGESRRPILYVGGGCAASGEELRRFVELTGIPVTTTLMGLGNFPSDDPLSLRMLGMHGTVYANYA
VDKADLLLAFGVRFDDRVTGKIEAFASRSKIVHIDIDPAEIGKNKQPHVSICADVKLALQGLNDLLN
GSKAQQGLDFGPWHKELDQQKREFPLGFKTFGEAIPPQYAIQVLDELTKGEAIIATGVGQHQMWA
AQYYTYKRPRQWLSSSGLGAMGFGLPAAAGAAVANPGVTVVDIDGDGSFLMNIQELALIRIENLP
VKVMILNNQHLGMVVQWEDRFYKANRAHTYLGNPENESEIYPDFVTIAKGFNVPAVRVTKKSEVT
AAIKKMLETPGPYLLDIIVPHQEHVLPMIPSGGAFKDMIMEGDGRTSY*
>SEQ ID NO:9 TaPPO-A amino acid sequence 
MLTSATAPSSSCSSHAPTRFASARGRRLRPVLAMAASDDPRARSVAVIGAGVSGLVAAYRLRKSGVR
VTVFEAEDRAGGKIRTNSDGGFLWDEGANTMTESALEASRLIDDLGLEDRLQYPNSQHKRYTVKD
GAPALIPSDPIALMKSTVLSTKSKFKFFLEPFLYEKSSTRNSKKVSDEHLRESVGSFFERHFGKEVVD
YLIDPFVAGTSAGDPESLSIRHAFPGLWNLEKKYGSLIVGAILSKLTAKGDSAKKGGTSSGKGRSKR
ASFSFHGGMQTLVDALHKEVGDSNVKLGTQVLSLACNCDELSASDGWSIFVDSKDASSKELAKN
QSFDAVIMTAPLSNVQRMKFTKGGAPFVLDFLPKVDYLPLSLMVTAFKKEDVKRPLEGFGVLIPFK
EQQKHGLKTLGTLFSSMMFPDRAPNDQYLFTTFIGGSHNRDLAGAPTPILKRFVTSDLTKLLGVKG
QPTFVKHIHWRNAFPLYGHDYDSALEAIGKMESDLPGFFYAGNNKDGLAVGNVIASGSNTADLVIS
YLESGIKQVS*
>SEQ ID NO:10 TaPPO-B amino acid sequence 
MLTSATAPPSSSSCSSHAPTRFASARGRRLRPVLAMAASDDPRARSVAVVGAGVSGLVAAYRLRKS
GVRVTVFEAEDRAGGKIRTNSDGGFLWDEGANTMTESALEASRLIDDLGLEDRLQYPNSQHKRYT
VKNGAPALIPSDPIALMKSTVLSTKSKFKLFLEPFLYEKSSTRNSKKVSDEHLRESVGSFFERHFGKE
VVDYLIDPFVAGTSAGDPESLSIRHAFPGLWNLEKKYGSLIVGAILSKLTAKGDSAKKGGASSGKGR
SKRASFSFHGGMQTLVDALHKEVGDNNVKLGTQVLSLACKCDELSASDGWSIFVDSKDATSKGL
AKNQSFDAVIMTAPLSNVQRMRFTKGGAPFVLDFLPKVDYLPLSLMVTAFKKEDVKRPLEGFGVLI
PFKEQQKHGLKTLGTLFSSMMFPDRAPNDQYLFTTFIGGSHNRDLAAAPTATLKQFVTSDLTKLLG
VEGQPTFVKHIHWRDAFPLYGHDYDSALEAIGKMESDLPGFFYAGNNKDGLAVGNVIASGSNTAD
LVISYLESGIKQAS*
>SEQ ID NO:11 TaPPO-D amino acid sequence 
MLTSATAPSSSSSCAPTRFASSCRPRRSASARGRRPVLAMAASDDPRARSVAVVGAGVSGLVAAYR
LRKSGVRVTVFEAEDRAGGKIRTNSDGGFLWDEGANTMTESALEASRLIDDLGLEDRLQYPNSQH
 
KRYTVKDGAPALIPSDPIALMKSTLLSTKSKFKLFLEPFLYEKSSTRNSKKVSDEHLRESVGSFFERH
FGREVVDYLIDPFVAGTSAGDPESLSIRHAFPGLWNLEKKYGSLIVGAILSKLTAKGDSSKKGGASS
GKGRSKRASFSFHGGMQTLVDALHKEVGDSNVKLGTQVLSLACNCDELSASDGWSIFVDSKDASS
KELAKNQSFDAVIMTAPLSNVQRMRFTKGGAPFVLDFLPKVDYLPLSLMVTAFKKEDVKRPLEGF
GVLIPFKEQQKHGLKTLGTLFSSMMFPDRAPNDQYLFTTFIGGSHNRDLAGAPTAILKQFVTSDLTK
LLGVVGQPTFVKHIHWRNAFPLYGHDYDSALEAIGKMESDLPGFFYAGNNKDGLAVGNVIASGSN
TADLVISYLESGIKQVS*
>SEQ ID NO:12 TaPPO-A-R127C 
MLTSATAPSSSCSSHAPTRFASARGRRLRPVLAMAASDDPRARSVAVIGAGVSGLVAAYRLRKSGVR
VTVFEAEDRAGGKIRTNSDGGFLWDEGANTMTESALEASRLIDDLGLEDRLQYPNSQHKCYTVKD
GAPALIPSDPIALMKSTVLSTKSKFKFFLEPFLYEKSSTRNSKKVSDEHLRESVGSFFERHFGKEVVD
YLIDPFVAGTSAGDPESLSIRHAFPGLWNLEKKYGSLIVGAILSKLTAKGDSAKKGGTSSGKGRSKR
ASFSFHGGMQTLVDALHKEVGDSNVKLGTQVLSLACNCDELSASDGWSIFVDSKDASSKELAKN
QSFDAVIMTAPLSNVQRMKFTKGGAPFVLDFLPKVDYLPLSLMVTAFKKEDVKRPLEGFGVLIPFK
EQQKHGLKTLGTLFSSMMFPDRAPNDQYLFTTFIGGSHNRDLAGAPTPILKRFVTSDLTKLLGVKG
QPTFVKHIHWRNAFPLYGHDYDSALEAIGKMESDLPGFFYAGNNKDGLAVGNVIASGSNTADLVIS
YLESGIKQVS*
>SEQ ID NO:13 TaPPO-A-R127G 
MLTSATAPSSSCSSHAPTRFASARGRRLRPVLAMAASDDPRARSVAVIGAGVSGLVAAYRLRKSGVR
VTVFEAEDRAGGKIRTNSDGGFLWDEGANTMTESALEASRLIDDLGLEDRLQYPNSQHKGYTVKD
GAPALIPSDPIALMKSTVLSTKSKFKFFLEPFLYEKSSTRNSKKVSDEHLRESVGSFFERHFGKEVVD
YLIDPFVAGTSAGDPESLSIRHAFPGLWNLEKKYGSLIVGAILSKLTAKGDSAKKGGTSSGKGRSKR
ASFSFHGGMQTLVDALHKEVGDSNVKLGTQVLSLACNCDELSASDGWSIFVDSKDASSKELAKN
QSFDAVIMTAPLSNVQRMKFTKGGAPFVLDFLPKVDYLPLSLMVTAFKKEDVKRPLEGFGVLIPFK
EQQKHGLKTLGTLFSSMMFPDRAPNDQYLFTTFIGGSHNRDLAGAPTPILKRFVTSDLTKLLGVKG
QPTFVKHIHWRNAFPLYGHDYDSALEAIGKMESDLPGFFYAGNNKDGLAVGNVIASGSNTADLVIS
YLESGIKQVS*
>SEQ ID NO:14 TaPPO-B-R129C 
MLTSATAPPSSSSCSSHAPTRFASARGRRLRPVLAMAASDDPRARSVAVVGAGVSGLVAAYRLRKS
GVRVTVFEAEDRAGGKIRTNSDGGFLWDEGANTMTESALEASRLIDDLGLEDRLQYPNSQHKCYT
VKNGAPALIPSDPIALMKSTVLSTKSKFKLFLEPFLYEKSSTRNSKKVSDEHLRESVGSFFERHFGKE
VVDYLIDPFVAGTSAGDPESLSIRHAFPGLWNLEKKYGSLIVGAILSKLTAKGDSAKKGGASSGKGR
SKRASFSFHGGMQTLVDALHKEVGDNNVKLGTQVLSLACKCDELSASDGWSIFVDSKDATSKGL
AKNQSFDAVIMTAPLSNVQRMRFTKGGAPFVLDFLPKVDYLPLSLMVTAFKKEDVKRPLEGFGVLI
PFKEQQKHGLKTLGTLFSSMMFPDRAPNDQYLFTTFIGGSHNRDLAAAPTATLKQFVTSDLTKLLG
VEGQPTFVKHIHWRDAFPLYGHDYDSALEAIGKMESDLPGFFYAGNNKDGLAVGNVIASGSNTAD
LVISYLESGIKQAS*
>SEQ ID NO:15 TaPPO-D-R133C 
MLTSATAPSSSSSCAPTRFASSCRPRRSASARGRRPVLAMAASDDPRARSVAVVGAGVSGLVAAYR
LRKSGVRVTVFEAEDRAGGKIRTNSDGGFLWDEGANTMTESALEASRLIDDLGLEDRLQYPNSQH
KCYTVKDGAPALIPSDPIALMKSTLLSTKSKFKLFLEPFLYEKSSTRNSKKVSDEHLRESVGSFFERH
FGREVVDYLIDPFVAGTSAGDPESLSIRHAFPGLWNLEKKYGSLIVGAILSKLTAKGDSSKKGGASS
GKGRSKRASFSFHGGMQTLVDALHKEVGDSNVKLGTQVLSLACNCDELSASDGWSIFVDSKDASS
KELAKNQSFDAVIMTAPLSNVQRMRFTKGGAPFVLDFLPKVDYLPLSLMVTAFKKEDVKRPLEGF
GVLIPFKEQQKHGLKTLGTLFSSMMFPDRAPNDQYLFTTFIGGSHNRDLAGAPTAILKQFVTSDLTK
LLGVVGQPTFVKHIHWRNAFPLYGHDYDSALEAIGKMESDLPGFFYAGNNKDGLAVGNVIASGSN
TADLVISYLESGIKQVS*
>SEQ ID NO:16 TaPPO-D-R133C/T135I 
MLTSATAPSSSSSCAPTRFASSCRPRRSASARGRRPVLAMAASDDPRARSVAVVGAGVSGLVAAYR
LRKSGVRVTVFEAEDRAGGKIRTNSDGGFLWDEGANTMTESALEASRLIDDLGLEDRLQYPNSQH
KCYIVKDGAPALIPSDPIALMKSTLLSTKSKFKLFLEPFLYEKSSTRNSKKVSDEHLRESVGSFFERH
FGREVVDYLIDPFVAGTSAGDPESLSIRHAFPGLWNLEKKYGSLIVGAILSKLTAKGDSSKKGGASS
GKGRSKRASFSFHGGMQTLVDALHKEVGDSNVKLGTQVLSLACNCDELSASDGWSIFVDSKDASS
KELAKNQSFDAVIMTAPLSNVQRMRFTKGGAPFVLDFLPKVDYLPLSLMVTAFKKEDVKRPLEGF
GVLIPFKEQQKHGLKTLGTLFSSMMFPDRAPNDQYLFTTFIGGSHNRDLAGAPTAILKQFVTSDLTK
 
LLGVVGQPTFVKHIHWRNAFPLYGHDYDSALEAIGKMESDLPGFFYAGNNKDGLAVGNVIASGSN
TADLVISYLESGIKQVS*
>SEQ ID NO:17 wildtype spCas9 amino acid sequence
>SEQ ID NO:18 nCas9 (D10A) amino acid sequence 
MDKKYSIGLAIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVAYHEKYPT
IYHLRKKLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGM
RKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEIRKRPL
IETNGETGEIVWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGD
>SEQ ID NO:19 nCas9-NG(D10A) amino acid sequence 
MDKKYSIGLAIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVAYHEKYPT
IYHLRKKLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGM
RKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLI
 
NGIRDKQSGKTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEIRKRPL
IETNGETGEIVWDKGRDFATVRKVLSMPQVNIVKKTEVQTGGFSKESIRPKRNSDKLIARKKDWDP
KKYGGFVSPTVAYSVLVVAKVEKGKSKKLKSVKELLGITIMERSSFEKNPIDFLEAKGYKEVKKDLI
IKLPKYSLFELENGRKRMLASARFLQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPRAFKYFDT
TIDRKVYRSTKEVLDATLIHQSITGLYETRIDLSQLGGD
>SEQ ID NO:20 nCas9-VQR(D10A) amino acid sequence 
MDKKYSIGLAIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVAYHEKYPT
IYHLRKKLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGM
RKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEIRKRPL
IETNGETGEIVWDKGRDFATVRKVLSMPQVNIVKKTEVQTGGFSKESILPKRNSDKLIARKKDWDP
KKYGGFVSPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFD
TTIDRKQYRSTKEVLDATLIHQSITGLYETRIDLSQLGGD
>SEQ ID NO:21 A APOBEC3A amino acid sequence 
MEASPASGPRHLMDPHIFTSNFNNGIGRHKTYLCYEVERLDNGTSVKMDQHRGFLHNQAKNLLC
GFYGRHAELRFLDLVPSLQLDPAQIYRVTWFISWSPCFSWGCAGEVRAFLQENTHVRLRIFAARIYD
YDPLYKEALQMLRDAGAQVSIMTYDEFKHCWDTFVDHQGCPFQPWDGLDEHSQALSGRLRAILQ
NQGN
>SEQ ID NO:22 PmCDA1 amino acid sequence 
MTDAEYVRIHEKLDIYTFKKQFFNNKKSVSHRCYVLFELKRRGERRACFWGYAVNKPQSGTERGI
HAEIFSIRKVEEYLRDNPGQFTINWYSSWSPCADCAEKILEWYNQELRGNGHTLKIWACKLYYEKN
ARNQIGLWNLRDNGVGLNVMVSEHYQCCRKIFIQSSHNQLNENRWLEKTLKRAEKRRSELSIMIQ
VKILHTTKSPAVSGSETPGTSESATPESLKMAPKKKRKVGIHGVPAA
>SEQ ID NO:23 ecTadA7.10 amino acid sequence 
MSEVEFSHEYWMRHALTLAKRARDEREVPVGAVLVLNNRVIGEGWNRAIGLHDPTAHAEIMALR
QGGLVMQNYRLIDATLYVTFEPCVMCAGAMIHSRIGRVVFGVRNAKTGAAGSLMDVLHYPGMNH
RVEITEGILADECAALLCYFFRMPRQVFNAQKKAQSSTD
>SEQ ID NO:24 PmCDA1-VQR-PBE amino acid sequence 
MLTLLFGVTSAKLVDGSMPKKKRKVMTDAEYVRIHEKLDIYTFKKQFFNNKKSVSHRCYVLFELK
RRGERRACFWGYAVNKPQSGTERGIHAEIFSIRKVEEYLRDNPGQFTINWYSSWSPCADCAEKILE
WYNQELRGNGHTLKIWACKLYYEKNARNQIGLWNLRDNGVGLNVMVSEHYQCCRKIFIQSSHNQ
LNENRWLEKTLKRAEKRRSELSIMIQVKILHTTKSPAVSGSETPGTSESATPESLKMAPKKKRKVGI
HGVPAADKKYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNIVDEVAYH
EKYPTIYHLRKKLVDSTDKADLRLIYLALAHMIKFRGHFLIEGDLNPDNSDVDKLFIQLVQTYNQLF
 
EENPINASGVDAKAILSARLSKSRRLENLIAQLPGEKKNGLFGNLIALSLGLTPNFKSNFDLAEDAK
LQLSKDTYDDDLDNLLAQIGDQYADLFLAAKNLSDAILLSDILRVNTEITKAPLSASMIKRYDEHH
QDLTLLKALVRQQLPEKYKEIFFDQSKNGYAGYIDGGASQEEFYKFIKPILEKMDGTEELLVKLNRE
DLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNASLGTYH
DLLKIIKDKDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEEGIKELGSQ
ILKEHPVENTQLQNEKLYLYYLQNGRDMYVDQELDINRLSDYDVDHIVPQSFLKDDSIDNKVLTRS
DKNRGKSDNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHHAHDAYLN
AVVGTALIKKYPKLESEFVYGDYKVYDVRKMIAKSEQEIGKATAKYFFYSNIMNFFKTEITLANGEI
RKRPLIETNGETGEIVWDKGRDFATVRKVLSMPQVNIVKKTEVQTGGFSKESILPKRNSDKLIARK
KDWDPKKYGGFVSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLKGSPEDNEQK
QLFVEQHKHYLDEIIEQISEFSKRVILADANLDKVLSAYNKHRDKPIREQAENIIHLFTLTNLGAPAA
FKYFDTTIDRKQYRSTKEVLDATLIHQSITGLYETRIDLSQLGGDKRPAATKKAGQAKKKKTRDSG
GSTNLSDIIEKETGKQLVIQESILMLPEEVEEVIGNKPESDILVHTAYDESTDENVMLLTSDAPEYKP
WALVIQDSNGENKIKMLSGGSPKKKRKV
>SEQ ID NO:25 A3A-PBE amino acid sequence 
LTLLFGVTSAKLVDGSMEASPASGPRHLMDPHIFTSNFNNGIGRHKTYLCYEVERLDNGTSVKMD
QHRGFLHNQAKNLLCGFYGRHAELRFLDLVPSLQLDPAQIYRVTWFISWSPCFSWGCAGEVRAFL
QENTHVRLRIFAARIYDYDPLYKEALQMLRDAGAQVSIMTYDEFKHCWDTFVDHQGCPFQPWDG
LDEHSQALSGRLRAILQNQGNSGSETPGTSESATPESLKDKKYSIGLAIGTNSVGWAVITDEYKVPS
KKFKVLGNTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIYLALAHMI
KFRGHFLIEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQSKNGYAGYI
DGGASQEEFYKFIKPILEKMDGTEELLVKLNREDLLRKQRTFDNGSIPHQIHLGELHAILRRQEDFY
PFLKDNREKIEKILTFRIPYYVGPLARGNSRFAWMTRKSEETITPWNFEEVVDKGASAQSFIERMTN
FDKNLPNEKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRDMYVDQ
ELDINRLSDYDVDHIVPQSFLKDDSIDNKVLTRSDKNRGKSDNVPSEEVVKKMKNYWRQLLNAKL
ITQRKFDNLTKAERGGLSELDKAGFIKRQLVETRQITKHVAQILDSRMNTKYDENDKLIREVKVITL
KSKLVSDFRKDFQFYKVREINNYHHAHDAYLNAVVGTALIKKYPKLESEFVYGDYKVYDVRKMIA
KSEQEIGKATAKYFFYSNIMNFFKTEITLANGEIRKRPLIETNGETGEIVWDKGRDFATVRKVLSMP
QVNIVKKTEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIHQSITGLYET
RIDLSQLGGDKRPAATKKAGQAKKKKTRDSGGSTNLSDIIEKETGKQLVIQESILMLPEEVEEVIGN
KPESDILVHTAYDESTDENVMLLTSDAPEYKPWALVIQDSNGENKIKMLSGGSPKKKRKV
>SEQ ID NO:26 B genome ALS target sequence
   CCTACCCTGGCGGCGCGTCC
>SEQ ID NO:27 D genome ALS target sequence
   CCTACCCCGGCGGCGCCTCC
>SEQ ID NO:28 A genome PPO target sequence
   AAGCGTTATACTGTTAAGGA
>SEQ ID NO:29 B genome PPO target sequence
   AAGCGTTATACTGTTAAGAA
>SEQ ID NO:30 D genome PPO target sequence
   AAGCGTTATACTGTTAAGGA
>SEQ ID NO:31 Rice ACCase 
MPMRPWEFIILGRTAPISPPPLGISAFSRPLRLRLRLRLAAVVDAARIQTPPRVAFSAFFFFFFFFSEGL
SREGTAARLPALIYRVPFIPCLLQLQIRAPHADPRPAGFSCAHHFLALAQGLSSSLLSGTISHIWGFIFS
LYGTTHLRNRAILLFGLAGIIDLPNDAASEVDISHGSEDPRGPTVPGSYQMNGIINETHNGRHASVS
KVVEFCTALGGKTPIHSVLVANNGMAAAKFMRSVRTWANDTFGSEKAIQLIAMATPEDLRINAEHI
RIADQFVEVPGGTNNNNYANVQLIVEIAERTGVSAVWPGWGHASENPELPDALTAKGIVFLGPPAS
SMHALGDKVGSALIAQAAGVPTLAWSGSHVEVPLECCLDSIPDEMYRKACVTTTEEAVASCQVVG
YPAMIKASWGGGGKGIRKVHNDDEVRTLFKQVQGEVPGSPIFIMRLAAQSRHLEVQLLCDQYGNV
AALHSRDCSVQRRHQKIIEEGPVTVAPRETVKELEQAARRLAKAVGYVGAATVEYLYSMETGEYY
FLELNPRLQVEHPVTEWIAEVNLPAAQVAVGMGIPLWQIPEIRRFYGMNHGGGYDLWRKTAALAT
PFNFDEVDSKWPKGHCVAVRITSEDPDDGFKPTGGKVKEISFKSKPNVWAYFSVKSGGGIHEFADS
QFGHVFAYGTTRSAAITTMALALKEVQIRGEIHSNVDYTVDLLNASDFRENKIHTGWLDTRIAMRV
QAERPPWYISVVGGALYKTVTANTATVSDYVGYLTKGQIPPKHISLVYTTVALNIDGKKYTIDTVRS
GHGSYRLRMNGSTVDANVQILCDGGLLMQLDGNSHVIYAEEEASGTRLLIDGKTCMLQNDHDPS
KLLAETPCKLLRFLVADGAHVDADVPYAEVEVMKMCMPLLSPASGVIHVVMSEGQAMQAGDLIA
RLDLDDPSAVKRAEPFEDTFPQMGLPIAASGQVHKLCAASLNACRMILAGYEHDIDKVVPELVYCL
DTPELPFLQWEELMSVLATRLPRNLKSELEGKYEEYKVKFDSGIINDFPANMLRVIIEENLACGSEK
EKATNERLVEPLMSLLKSYEGGRESHAHFVVKSLFEEYLYVEELFSDGIQSDVIERLRLQHSKDLQK
VVDIVLSHQSVRNKTKLILKLMESLVYPNPAAYRDQLIRFSSLNHKAYYKLALKASELLEQTKLSEL
RARIARSLSELEMFTEESKGLSMHKREIAIKESMEDLVTAPLPVEDALISLFDCSDTTVQQRVIETYI
ARLYQPHLVKDSIKMKWIESGVIALWEFPEGHFDARNGGAVLGDKRWGAMVIVKSLESLSMAIRF
ALKETSHYTSSEGNMMHIALLGADNKMHIIQESGDDADRIAKLPLILKDNVTDLHASGVKTISFIV
QRDEARMTMRRTFLWSDEKLSYEEEPILRHVEPPLSALLELDKLKVKGYNEMKYTPSRDRQWHIY
TLRNTENPKMLHRVFFRTLVRQPSVSNKFSSGQIGDMEVGSAEEPLSFTSTSILRSLMTAIEELELHAI
RTGHSHMYLHVLKEQKLLDLVPVSGNTVLDVGQDEATAYSLLKEMAMKIHELVGARMHHLSVCQ
WEVKLKLDCDGPASGTWRIVTTNVTSHTCTVDIYREMEDKESRKLVYHPATPAAGPLHGVALNNP
YQPLSVIDLKRCSARNNRTTYCYDFPLAFETAVRKSWSSSTSGASKGVENAQCYVKATELVFADKH
GSWGTPLVQMDRPAGLNDIGMVAWTLKMSTPEFPSGREIIVVANDITFRAGSFGPREDAFFEAVTNL
ACEKKLPLIYLAANSGARIGIADEVKSCFRVGWSDDGSPERGFQYIYLSEEDYARIGTSVIAHKMQL
DSGEIRWVIDSVVGKEDGLGVENIHGSAAIASAYSRAYKETFTLTFVTGRTVGIGAYLARLGIRCIQR
LDQPIILTGYSALNKLLGREVYSSHMQLGGPKIMATNGVVHLTVSDDLEGVSNILRWLSYVPAYIGG
PLPVTTPLDPPDRPVAYIPENSCDPRAAIRGVDDSQGKWLGGMFDKDSFVETFEGWAKTVVTGRA
KLGGIPVGVIAVETQTMMQTIPADPGQLDSREQSVPRAGQVWFPDSATKTAQALLDFNREGLPLFIL
ANWRGFSGGQRDLFEGILQAGSTIVENLRTYNQPAFVYIPMAAELRGGAWVVVDSKINPDRIECYA
ERTAKGNVLEPQGLIEIKFRSEELQDCMSRLDPTLIDLKAKLEVANKNGSADTKSLQENIEARTKQL
MPLYTQIAIRFAELHDTSLRMAAKGVIKKVVDWEESRSFFYKRLRRRISEDVLAKEIRAVAGEQFSH
QPAIELIKKWYSASHAAEWDDDDAFVAWMDNPENYKDYIQYLKAQRVSQSLSSLSDSSSDLQALP
QGLSMLLDKMDPSRRAQLVEEIRKVLG*
>SEQ ID NO:32 rice ACCase resistant mutant S1866F 
MPMRPWEFIILGRTAPISPPPLGISAFSRPLRLRLRLRLAAVVDAARIQTPPRVAFSAFFFFFFFFSEGL
SREGTAARLPALIYRVPFIPCLLQLQIRAPHADPRPAGFSCAHHFLALAQGLSSSLLSGTISHIWGFIFS
LYGTTHLRNRAILLFGLAGIIDLPNDAASEVDISHGSEDPRGPTVPGSYQMNGIINETHNGRHASVS
KVVEFCTALGGKTPIHSVLVANNGMAAAKFMRSVRTWANDTFGSEKAIQLIAMATPEDLRINAEHI
RIADQFVEVPGGTNNNNYANVQLIVEIAERTGVSAVWPGWGHASENPELPDALTAKGIVFLGPPAS
SMHALGDKVGSALIAQAAGVPTLAWSGSHVEVPLECCLDSIPDEMYRKACVTTTEEAVASCQVVG
YPAMIKASWGGGGKGIRKVHNDDEVRTLFKQVQGEVPGSPIFIMRLAAQSRHLEVQLLCDQYGNV
AALHSRDCSVQRRHQKIIEEGPVTVAPRETVKELEQAARRLAKAVGYVGAATVEYLYSMETGEYY
FLELNPRLQVEHPVTEWIAEVNLPAAQVAVGMGIPLWQIPEIRRFYGMNHGGGYDLWRKTAALAT
PFNFDEVDSKWPKGHCVAVRITSEDPDDGFKPTGGKVKEISFKSKPNVWAYFSVKSGGGIHEFADS
QFGHVFAYGTTRSAAITTMALALKEVQIRGEIHSNVDYTVDLLNASDFRENKIHTGWLDTRIAMRV
QAERPPWYISVVGGALYKTVTANTATVSDYVGYLTKGQIPPKHISLVYTTVALNIDGKKYTIDTVRS
GHGSYRLRMNGSTVDANVQILCDGGLLMQLDGNSHVIYAEEEASGTRLLIDGKTCMLQNDHDPS
KLLAETPCKLLRFLVADGAHVDADVPYAEVEVMKMCMPLLSPASGVIHVVMSEGQAMQAGDLIA
RLDLDDPSAVKRAEPFEDTFPQMGLPIAASGQVHKLCAASLNACRMILAGYEHDIDKVVPELVYCL
 
DTPELPFLQWEELMSVLATRLPRNLKSELEGKYEEYKVKFDSGIINDFPANMLRVIIEENLACGSEK
EKATNERLVEPLMSLLKSYEGGRESHAHFVVKSLFEEYLYVEELFSDGIQSDVIERLRLQHSKDLQK
VVDIVLSHQSVRNKTKLILKLMESLVYPNPAAYRDQLIRFSSLNHKAYYKLALKASELLEQTKLSEL
RARIARSLSELEMFTEESKGLSMHKREIAIKESMEDLVTAPLPVEDALISLFDCSDTTVQQRVIETYI
ARLYQPHLVKDSIKMKWIESGVIALWEFPEGHFDARNGGAVLGDKRWGAMVIVKSLESLSMAIRF
ALKETSHYTSSEGNMMHIALLGADNKMHIIQESGDDADRIAKLPLILKDNVTDLHASGVKTISFIV
QRDEARMTMRRTFLWSDEKLSYEEEPILRHVEPPLSALLELDKLKVKGYNEMKYTPSRDRQWHIY
TLRNTENPKMLHRVFFRTLVRQPSVSNKFSSGQIGDMEVGSAEEPLSFTSTSILRSLMTAIEELELHAI
RTGHSHMYLHVLKEQKLLDLVPVSGNTVLDVGQDEATAYSLLKEMAMKIHELVGARMHHLSVCQ
WEVKLKLDCDGPASGTWRIVTTNVTSHTCTVDIYREMEDKESRKLVYHPATPAAGPLHGVALNNP
YQPLSVIDLKRCSARNNRTTYCYDFPLAFETAVRKSWSSSTSGASKGVENAQCYVKATELVFADKH
GSWGTPLVQMDRPAGLNDIGMVAWTLKMSTPEFPSGREIIVVANDITFRAGSFGPREDAFFEAVTNL
ACEKKLPLIYLAANSGARIGIADEVKSCFRVGWSDDGSPERGFQYIYLSEEDYARIGTSVIAHKMQL
DSGEIRWVIDFVVGKEDGLGVENIHGSAAIASAYSRAYKETFTLTFVTGRTVGIGAYLARLGIRCIQR
LDQPIILTGYSALNKLLGREVYSSHMQLGGPKIMATNGVVHLTVSDDLEGVSNILRWLSYVPAYIGG
PLPVTTPLDPPDRPVAYIPENSCDPRAAIRGVDDSQGKWLGGMFDKDSFVETFEGWAKTVVTGRA
KLGGIPVGVIAVETQTMMQTIPADPGQLDSREQSVPRAGQVWFPDSATKTAQALLDFNREGLPLFIL
ANWRGFSGGQRDLFEGILQAGSTIVENLRTYNQPAFVYIPMAAELRGGAWVVVDSKINPDRIECYA
ERTAKGNVLEPQGLIEIKFRSEELQDCMSRLDPTLIDLKAKLEVANKNGSADTKSLQENIEARTKQL
MPLYTQIAIRFAELHDTSLRMAAKGVIKKVVDWEESRSFFYKRLRRRISEDVLAKEIRAVAGEQFSH
QPAIELIKKWYSASHAAEWDDDDAFVAWMDNPENYKDYIQYLKAQRVSQSLSSLSDSSSDLQALP
QGLSMLLDKMDPSRRAQLVEEIRKVLG*
>SEQ ID NO:33 rice ACCase resistant mutant A1884P 
MPMRPWEFIILGRTAPISPPPLGISAFSRPLRLRLRLRLAAVVDAARIQTPPRVAFSAFFFFFFFFSEGL
SREGTAARLPALIYRVPFIPCLLQLQIRAPHADPRPAGFSCAHHFLALAQGLSSSLLSGTISHIWGFIFS
LYGTTHLRNRAILLFGLAGIIDLPNDAASEVDISHGSEDPRGPTVPGSYQMNGIINETHNGRHASVS
KVVEFCTALGGKTPIHSVLVANNGMAAAKFMRSVRTWANDTFGSEKAIQLIAMATPEDLRINAEHI
RIADQFVEVPGGTNNNNYANVQLIVEIAERTGVSAVWPGWGHASENPELPDALTAKGIVFLGPPAS
SMHALGDKVGSALIAQAAGVPTLAWSGSHVEVPLECCLDSIPDEMYRKACVTTTEEAVASCQVVG
YPAMIKASWGGGGKGIRKVHNDDEVRTLFKQVQGEVPGSPIFIMRLAAQSRHLEVQLLCDQYGNV
AALHSRDCSVQRRHQKIIEEGPVTVAPRETVKELEQAARRLAKAVGYVGAATVEYLYSMETGEYY
FLELNPRLQVEHPVTEWIAEVNLPAAQVAVGMGIPLWQIPEIRRFYGMNHGGGYDLWRKTAALAT
PFNFDEVDSKWPKGHCVAVRITSEDPDDGFKPTGGKVKEISFKSKPNVWAYFSVKSGGGIHEFADS
QFGHVFAYGTTRSAAITTMALALKEVQIRGEIHSNVDYTVDLLNASDFRENKIHTGWLDTRIAMRV
QAERPPWYISVVGGALYKTVTANTATVSDYVGYLTKGQIPPKHISLVYTTVALNIDGKKYTIDTVRS
GHGSYRLRMNGSTVDANVQILCDGGLLMQLDGNSHVIYAEEEASGTRLLIDGKTCMLQNDHDPS
KLLAETPCKLLRFLVADGAHVDADVPYAEVEVMKMCMPLLSPASGVIHVVMSEGQAMQAGDLIA
RLDLDDPSAVKRAEPFEDTFPQMGLPIAASGQVHKLCAASLNACRMILAGYEHDIDKVVPELVYCL
DTPELPFLQWEELMSVLATRLPRNLKSELEGKYEEYKVKFDSGIINDFPANMLRVIIEENLACGSEK
EKATNERLVEPLMSLLKSYEGGRESHAHFVVKSLFEEYLYVEELFSDGIQSDVIERLRLQHSKDLQK
VVDIVLSHQSVRNKTKLILKLMESLVYPNPAAYRDQLIRFSSLNHKAYYKLALKASELLEQTKLSEL
RARIARSLSELEMFTEESKGLSMHKREIAIKESMEDLVTAPLPVEDALISLFDCSDTTVQQRVIETYI
ARLYQPHLVKDSIKMKWIESGVIALWEFPEGHFDARNGGAVLGDKRWGAMVIVKSLESLSMAIRF
ALKETSHYTSSEGNMMHIALLGADNKMHIIQESGDDADRIAKLPLILKDNVTDLHASGVKTISFIV
QRDEARMTMRRTFLWSDEKLSYEEEPILRHVEPPLSALLELDKLKVKGYNEMKYTPSRDRQWHIY
TLRNTENPKMLHRVFFRTLVRQPSVSNKFSSGQIGDMEVGSAEEPLSFTSTSILRSLMTAIEELELHAI
RTGHSHMYLHVLKEQKLLDLVPVSGNTVLDVGQDEATAYSLLKEMAMKIHELVGARMHHLSVCQ
WEVKLKLDCDGPASGTWRIVTTNVTSHTCTVDIYREMEDKESRKLVYHPATPAAGPLHGVALNNP
YQPLSVIDLKRCSARNNRTTYCYDFPLAFETAVRKSWSSSTSGASKGVENAQCYVKATELVFADKH
GSWGTPLVQMDRPAGLNDIGMVAWTLKMSTPEFPSGREIIVVANDITFRAGSFGPREDAFFEAVTNL
ACEKKLPLIYLAANSGARIGIADEVKSCFRVGWSDDGSPERGFQYIYLSEEDYARIGTSVIAHKMQL
DSGEIRWVIDSVVGKEDGLGVENIHGSAPIASAYSRAYKETFTLTFVTGRTVGIGAYLARLGIRCIQR
LDQPIILTGYSALNKLLGREVYSSHMQLGGPKIMATNGVVHLTVSDDLEGVSNILRWLSYVPAYIGG
PLPVTTPLDPPDRPVAYIPENSCDPRAAIRGVDDSQGKWLGGMFDKDSFVETFEGWAKTVVTGRA
KLGGIPVGVIAVETQTMMQTIPADPGQLDSREQSVPRAGQVWFPDSATKTAQALLDFNREGLPLFIL
ANWRGFSGGQRDLFEGILQAGSTIVENLRTYNQPAFVYIPMAAELRGGAWVVVDSKINPDRIECYA
ERTAKGNVLEPQGLIEIKFRSEELQDCMSRLDPTLIDLKAKLEVANKNGSADTKSLQENIEARTKQL
MPLYTQIAIRFAELHDTSLRMAAKGVIKKVVDWEESRSFFYKRLRRRISEDVLAKEIRAVAGEQFSH
 
QPAIELIKKWYSASHAAEWDDDDAFVAWMDNPENYKDYIQYLKAQRVSQLSSLSDSSSDLQALP
QGLSMLLDKMDPSRRAQLVEEIRKVLG*
>SEQ ID NO:34 rice ACCase resistant mutant P1927F 
MPMRPWEFIILGRTAPISPPPLGISAFSRPLRLRLRLRLAAVVDAARIQTPPRVAFSAFFFFFFFSEGL
SREGTAARLPALIYRVPFIPCLLQLQIRAPHADPRPAGFSCAHHFLALAQGLSSSLLSGTISHIWGFIFS
LYGTTHLRNRAILLFGLAGIIDLPNDAASEVDISHGSEDPRGPTVPGSYQMNGIINETHNGRHASVS
KVVEFCTALGGKTPIHSVLVANNGMAAAKFMRSVRTWANDTFGSEKAIQLIAMATPEDLRINAEHI
RIADQFVEVPGGTNNNNYANVQLIVEIAERTGVSAVWPGWGHASENPELPDALTAKGIVFLGPPAS
SMHALGDKVGSALIAQAAGVPTLAWSGSHVEVPLECCLDSIPDEMYRKACVTTTEEAVASCQVVG
YPAMIKASWGGGGKGIRKVHNDDEVRTLFKQVQGEVPGSPIFIMRLAAQSRHLEVQLLCDQYGNV
AALHSRDCSVQRRHQKIIEEGPVTVAPRETVKELEQAARRLAKAVGYVGAATVEYLYSMETGEYY
FLELNPRLQVEHPVTEWIAEVNLPAAQVAVGMGIPLWQIPEIRRFYGMNHGGGYDLWRKTAALAT
PFNFDEVDSKWPKGHCVAVRITSEDPDDGFKPTGGKVKEISFKSKPNVWAYFSVKSGGGIHEFADS
QFGHVFAYGTTRSAAITTMALALKEVQIRGEIHSNVDYTVDLLNASDFRENKIHTGWLDTRIAMRV
QAERPPWYISVVGGALYKTVTANTATVSDYVGYLTKGQIPPKHISLVYTTVALNIDGKKYTIDTVRS
GHGSYRLRMNGSTVDANVQILCDGGLLMQLDGNSHVIYAEEEASGTRLLIDGKTCMLQNDHDPS
KLLAETPCKLLRFLVADGAHVDADVPYAEVEVMKMCMPLLSPASGVIHVVMSEGQAMQAGDLIA
RLDLDDPSAVKRAEPFEDTFPQMGLPIAASGQVHKLCAASLNACRMILAGYEHDIDKVVPELVYCL
DTPELPFLQWEELMSVLATRLPRNLKSELEGKYEEYKVKFDSGIINDFPANMLRVIIEENLACGSEK
EKATNERLVEPLMSLLKSYEGGRESHAHFVVKSLFEEYLYVEELFSDGIQSDVIERLRLQHSKDLQK
VVDIVLSHQSVRNKTKLILKLMESLVYPNPAAYRDQLIRFSSLNHKAYYKLALKASELLEQTKLSEL
RARIARSLSELEMFTEESKGLSMHKREIAIKESMEDLVTAPLPVEDALISLFDCSDTTVQQRVIETYI
ARLYQPHLVKDSIKMKWIESGVIALWEFPEGHFDARNGGAVLGDKRWGAMVIVKSLESLSMAIRF
ALKETSHYTSSEGNMMHIALLGADNKMHIIQESGDDADRIAKLPLILKDNVTDLHASGVKTISFIV
QRDEARMTMRRTFLWSDEKLSYEEEPILRHVEPPLSALLELDKLKVKGYNEMKYTPSRDRQWHIY
TLRNTENPKMLHRVFFRTLVRQPSVSNKFSSGQIGDMEVGSAEEPLSFTSTSILRSLMTAIEELELHAI
RTGHSHMYLHVLKEQKLLDLVPVSGNTVLDVGQDEATAYSLLKEMAMKIHELVGARMHHLSVCQ
WEVKLKLDCDGPASGTWRIVTTNVTSHTCTVDIYREMEDKESRKLVYHPATPAAGPLHGVALNNP
YQPLSVIDLKRCSARNNRTTYCYDFPLAFETAVRKSWSSSTSGASKGVENAQCYVKATELVFADKH
GSWGTPLVQMDRPAGLNDIGMVAWTLKMSTPEFPSGREIIVVANDITFRAGSFGPREDAFFEAVTNL
ACEKKLPLIYLAANSGARIGIADEVKSCFRVGWSDDGSPERGFQYIYLSEEDYARIGTSVIAHKMQL
DSGEIRWVIDSVVGKEDGLGVENIHGSAAIASAYSRAYKETFTLTFVTGRTVGIGAYLARLGIRCIQR
LDQFIILTGYSALNKLLGREVYSSHMQLGGPKIMATNGVVHLTVSDDLEGVSNILRWLSYVPAYIGG
PLPVTTPLDPPDRPVAYIPENSCDPRAAIRGVDDSQGKWLGGMFDKDSFVETFEGWAKTVVTGRA
KLGGIPVGVIAVETQTMMQTIPADPGQLDSREQSVPRAGQVWFPDSATKTAQALLDFNREGLPLFIL
ANWRGFSGGQRDLFEGILQAGSTIVENLRTYNQPAFVYIPMAAELRGGAWVVVDSKINPDRIECYA
ERTAKGNVLEPQGLIEIKFRSEELQDCMSRLDPTLIDLKAKLEVANKNGSADTKSLQENIEARTKQL
MPLYTQIAIRFAELHDTSLRMAAKGVIKKVVDWEESRSFFYKRLRRRISEDVLAKEIRAVAGEQFSH
QPAIELIKKWYSASHAAEWDDDDAFVAWMDNPENYKDYIQYLKAQRVSQSLSSLSDSSSDLQALP
QGLSMLLDKMDPSRRAQLVEEIRKVLG*
>SEQ ID NO:35 rice ACCase resistant mutant P2181L 
MPMRPWEFIILGRTAPISPPPLGISAFSRPLRLRLRLRLAAVVDAARIQTPPRVAFSAFFFFFFFFSEGL
SREGTAARLPALIYRVPFIPCLLQLQIRAPHADPRPAGFSCAHHFLALAQGLSSSLLSGTISHIWGFIFS
LYGTTHLRNRAILLFGLAGIIDLPNDAASEVDISHGSEDPRGPTVPGSYQMNGIINETHNGRHASVS
KVVEFCTALGGKTPIHSVLVANNGMAAAKFMRSVRTWANDTFGSEKAIQLIAMATPEDLRINAEHI
RIADQFVEVPGGTNNNNYANVQLIVEIAERTGVSAVWPGWGHASENPELPDALTAKGIVFLGPPAS
SMHALGDKVGSALIAQAAGVPTLAWSGSHVEVPLECCLDSIPDEMYRKACVTTTEEAVASCQVVG
YPAMIKASWGGGGKGIRKVHNDDEVRTLFKQVQGEVPGSPIFIMRLAAQSRHLEVQLLCDQYGNV
AALHSRDCSVQRRHQKIIEEGPVTVAPRETVKELEQAARRLAKAVGYVGAATVEYLYSMETGEYY
FLELNPRLQVEHPVTEWIAEVNLPAAQVAVGMGIPLWQIPEIRRFYGMNHGGGYDLWRKTAALAT
PFNFDEVDSKWPKGHCVAVRITSEDPDDGFKPTGGKVKEISFKSKPNVWAYFSVKSGGGIHEFADS
QFGHVFAYGTTRSAAITTMALALKEVQIRGEIHSNVDYTVDLLNASDFRENKIHTGWLDTRIAMRV
QAERPPWYISVVGGALYKTVTANTATVSDYVGYLTKGQIPPKHISLVYTTVALNIDGKKYTIDTVRS
GHGSYRLRMNGSTVDANVQILCDGGLLMQLDGNSHVIYAEEEASGTRLLIDGKTCMLQNDHDPS
KLLAETPCKLLRFLVADGAHVDADVPYAEVEVMKMCMPLLSPASGVIHVVMSEGQAMQAGDLIA
RLDLDDPSAVKRAEPFEDTFPQMGLPIAASGQVHKLCAASLNACRMILAGYEHDIDKVVPELVYCL
DTPELPFLQWEELMSVLATRLPRNLKSELEGKYEEYKVKFDSGIINDFPANMLRVIIEENLACGSEK
EKATNERLVEPLMSLLKSYEGGRESHAHFVVKSLFEEYLYVEELFSDGIQSDVIERLRLQHSKDLQK
 
VVDIVLSHQSVRNKTKLILKLMESLVYPNPAAYRDQLIRFSSLNHKAYYKLALKASELLEQTKLSEL
RARIARSLSELEMFTEESKGLSMHKREIAIKESMEDLVTAPLPVEDALISLFDCSDTTVQQRVIETYI
ARLYQPHLVKDSIKMKWIESGVIALWEFPEGHFDARNGGAVLGDKRWGAMVIVKSLESLSMAIRF
ALKETSHYTSSEGNMMHIALLGADNKMHIIQESGDDADRIAKLPLILKDNVTDLHASGVKTISFIV
QRDEARMTMRRTFLWSDEKLSYEEEPILRHVEPPLSALLELDKLKVKGYNEMKYTPSRDRQWHIY
TLRNTENPKMLHRVFFRTLVRQPSVSNKFSSGQIGDMEVGSAEEPLSFTSTSILRSLMTAIEELELHAI
RTGHSHMYLHVLKEQKLLDLVPVSGNTVLDVGQDEATAYSLLKEMAMKIHELVGARMHHLSVCQ
WEVKLKLDCDGPASGTWRIVTTNVTSHTCTVDIYREMEDKESRKLVYHPATPAAGPLHGVALNNP
YQPLSVIDLKRCSARNNRTTYCYDFPLAFETAVRKSWSSSTSGASKGVENAQCYVKATELVFADKH
GSWGTPLVQMDRPAGLNDIGMVAWTLKMSTPEFPSGREIIVVANDITFRAGSFGPREDAFFEAVTNL
ACEKKLPLIYLAANSGARIGIADEVKSCFRVGWSDDGSPERGFQYIYLSEEDYARIGTSVIAHKMQL
DSGEIRWVIDSVVGKEDGLGVENIHGSAAIASAYSRAYKETFTLTFVTGRTVGIGAYLARLGIRCIQR
LDQPIILTGYSALNKLLGREVYSSHMQLGGPKIMATNGVVHLTVSDDLEGVSNILRWLSYVPAYIGG
PLPVTTPLDPPDRPVAYIPENSCDPRAAIRGVDDSQGKWLGGMFDKDSFVETFEGWAKTVVTGRA
KLGGIPVGVIAVETQTMMQTIPADPGQLDSREQSVPRAGQVWFPDSATKTAQALLDFNREGLPLFIL
ANWRGFSGGQRDLFEGILQAGSTIVENLRTYNQPAFVYIPMAAELRGGAWVVVDSKINLDRIECYA
ERTAKGNVLEPQGLIEIKFRSEELQDCMSRLDPTLIDLKAKLEVANKNGSADTKSLQENIEARTKQL
MPLYTQIAIRFAELHDTSLRMAAKGVIKKVVDWEESRSFFYKRLRRRISEDVLAKEIRAVAGEQFSH
QPAIELIKKWYSASHAAEWDDDDAFVAWMDNPENYKDYIQYLKAQRVSQSLSSLSDSSSDLQALP
QGLSMLLDKMDPSRRAQLVEEIRKVLG*
>SEQ ID NO:36 amino acid sequence of CT domain of WT OsACC 
AFETAVRKSWSSSTSGASKGVENAQCYVKATELVFADKHGSWGTPLVQMDRPAGLNDIGMVAWT
LKMSTPEFPSGREIIVVANDITFRAGSFGPREDAFFEAVTNLACEKKLPLIYLAANSGARIGIADEVK
SCFRVGWSDDGSPERGFQYIYLSEEDYARIGTSVIAHKMQLDSGEIRWVIDSVVGKEDGLGVENIH
GSAAIASAYSRAYKETFTLTFVTGRTVGIGAYLARLGIRCIQRLDQPIILTGYSALNKLLGREVYSSH
MQLGGPKIMATNGVVHLTVSDDLEGVSNILRWLSYVPAYIGGPLPVTTPLDPPDRPVAYIPENSCDP
RAAIRGVDDSQGKWLGGMFDKDSFVETFEGWAKTVVTGRAKLGGIPVGVIAVETQTMMQTIPAD
PGQLDSREQSVPRAGQVWFPDSATKTAQALLDFNREGLPLFILANWRGFSGGQRDLFEGILQAGST
IVENLRTYNQPAFVYIPMAAELRGGAWVVVDSKINPDRIECYAERTAKGNVLEPQGLIEIKFRSEEL
QDCMSRLDPTLIDLKAKLEVANKNGSADTKSLQENIEARTKQLMPLYTQIAIRFAELHDTSLRMAA
KGVIKKVVDWEESRSFFYKRLRRRISEDVLAKEIRAVAGEQFSHQPAIELIKKWYSASHAAEWDDD
DAFVAWMDNPENYKDYIQYLKAQRVSQSLSSLSDSSSDLQALPQGLSML
>SEQ ID NO:37 amino acid sequence of CT domain of OsACC resistant mutant S1866F 
AFETAVRKSWSSSTSGASKGVENAQCYVKATELVFADKHGSWGTPLVQMDRPAGLNDIGMVAWT
LKMSTPEFPSGREIIVVANDITFRAGSFGPREDAFFEAVTNLACEKKLPLIYLAANSGARIGIADEVK
SCFRVGWSDDGSPERGFQYIYLSEEDYARIGTSVIAHKMQLDSGEIRWVIDFVVGKEDGLGVENIH
GSAAIASAYSRAYKETFTLTFVTGRTVGIGAYLARLGIRCIQRLDQPIILTGYSALNKLLGREVYSSH
MQLGGPKIMATNGVVHLTVSDDLEGVSNILRWLSYVPAYIGGPLPVTTPLDPPDRPVAYIPENSCDP
RAAIRGVDDSQGKWLGGMFDKDSFVETFEGWAKTVVTGRAKLGGIPVGVIAVETQTMMQTIPAD
PGQLDSREQSVPRAGQVWFPDSATKTAQALLDFNREGLPLFILANWRGFSGGQRDLFEGILQAGST
IVENLRTYNQPAFVYIPMAAELRGGAWVVVDSKINPDRIECYAERTAKGNVLEPQGLIEIKFRSEEL
QDCMSRLDPTLIDLKAKLEVANKNGSADTKSLQENIEARTKQLMPLYTQIAIRFAELHDTSLRMAA
KGVIKKVVDWEESRSFFYKRLRRRISEDVLAKEIRAVAGEQFSHQPAIELIKKWYSASHAAEWDDD
DAFVAWMDNPENYKDYIQYLKAQRVSQSLSSLSDSSSDLQALPQGLSML
>SEQ ID NO:38 amino acid sequence of CT domain of OsACC resistant mutant A1884P 
AFETAVRKSWSSSTSGASKGVENAQCYVKATELVFADKHGSWGTPLVQMDRPAGLNDIGMVAWT
LKMSTPEFPSGREIIVVANDITFRAGSFGPREDAFFEAVTNLACEKKLPLIYLAANSGARIGIADEVK
SCFRVGWSDDGSPERGFQYIYLSEEDYARIGTSVIAHKMQLDSGEIRWVIDSVVGKEDGLGVENIH
GSAPIASAYSRAYKETFTLTFVTGRTVGIGAYLARLGIRCIQRLDQPIILTGYSALNKLLGREVYSSH
MQLGGPKIMATNGVVHLTVSDDLEGVSNILRWLSYVPAYIGGPLPVTTPLDPPDRPVAYIPENSCDP
RAAIRGVDDSQGKWLGGMFDKDSFVETFEGWAKTVVTGRAKLGGIPVGVIAVETQTMMQTIPAD
PGQLDSREQSVPRAGQVWFPDSATKTAQALLDFNREGLPLFILANWRGFSGGQRDLFEGILQAGST
IVENLRTYNQPAFVYIPMAAELRGGAWVVVDSKINPDRIECYAERTAKGNVLEPQGLIEIKFRSEEL
QDCMSRLDPTLIDLKAKLEVANKNGSADTKSLQENIEARTKQLMPLYTQIAIRFAELHDTSLRMAA
KGVIKKVVDWEESRSFFYKRLRRRISEDVLAKEIRAVAGEQFSHQPAIELIKKWYSASHAAEWDDD
 DAFVAWMDNPENYKDYIQYLKAQRVSQSLSSLSDSSSDLQALPQGLSML
>SEQ ID NO:39 amino acid sequence of CT domain of OsACC resistant mutant P1927F 
AFETAVRKSWSSSTSGASKGVENAQCYVKATELVFADKHGSWGTPLVQMDRPAGLNDIGMVAWT
LKMSTPEFPSGREIIVVANDITFRAGSFGPREDAFFEAVTNLACEKKLPLIYLAANSGARIGIADEVK
SCFRVGWSDDGSPERGFQYIYLSEEDYARIGTSVIAHKMQLDSGEIRWVIDSVVGKEDGLGVENIH
GSAAIASAYSRAYKETFTLTFVTGRTVGIGAYLARLGIRCIQRLDQFIILTGYSALNKLLGREVYSSH
MQLGGPKIMATNGVVHLTVSDDLEGVSNILRWLSYVPAYIGGPLPVTTPLDPPDRPVAYIPENSCDP
RAAIRGVDDSQGKWLGGMFDKDSFVETFEGWAKTVVTGRAKLGGIPVGVIAVETQTMMQTIPAD
PGQLDSREQSVPRAGQVWFPDSATKTAQALLDFNREGLPLFILANWRGFSGGQRDLFEGILQAGST
IVENLRTYNQPAFVYIPMAAELRGGAWVVVDSKINPDRIECYAERTAKGNVLEPQGLIEIKFRSEEL
QDCMSRLDPTLIDLKAKLEVANKNGSADTKSLQENIEARTKQLMPLYTQIAIRFAELHDTSLRMAA
KGVIKKVVDWEESRSFFYKRLRRRISEDVLAKEIRAVAGEQFSHQPAIELIKKWYSASHAAEWDDD
DAFVAWMDNPENYKDYIQYLKAQRVSQSLSSLSDSSSDLQALPQGLSML
>SEQ ID NO:40 amino acid sequence of CT domain of OsACC resistant mutant P2181L 
AFETAVRKSWSSSTSGASKGVENAQCYVKATELVFADKHGSWGTPLVQMDRPAGLNDIGMVAWT
LKMSTPEFPSGREIIVVANDITFRAGSFGPREDAFFEAVTNLACEKKLPLIYLAANSGARIGIADEVK
SCFRVGWSDDGSPERGFQYIYLSEEDYARIGTSVIAHKMQLDSGEIRWVIDSVVGKEDGLGVENIH
GSAAIASAYSRAYKETFTLTFVTGRTVGIGAYLARLGIRCIQRLDQPIILTGYSALNKLLGREVYSSH
MQLGGPKIMATNGVVHLTVSDDLEGVSNILRWLSYVPAYIGGPLPVTTPLDPPDRPVAYIPENSCDP
RAAIRGVDDSQGKWLGGMFDKDSFVETFEGWAKTVVTGRAKLGGIPVGVIAVETQTMMQTIPAD
PGQLDSREQSVPRAGQVWFPDSATKTAQALLDFNREGLPLFILANWRGFSGGQRDLFEGILQAGST
IVENLRTYNQPAFVYIPMAAELRGGAWVVVDSKINLDRIECYAERTAKGNVLEPQGLIEIKFRSEEL
QDCMSRLDPTLIDLKAKLEVANKNGSADTKSLQENIEARTKQLMPLYTQIAIRFAELHDTSLRMAA
KGVIKKVVDWEESRSFFYKRLRRRISEDVLAKEIRAVAGEQFSHQPAIELIKKWYSASHAAEWDDD
DAFVAWMDNPENYKDYIQYLKAQRVSQSLSSLSDSSSDLQALPQGLSML
>SEQ ID NO:41 OsACC-S1866 target sequence
   ATTGATTCTGTTGTGGGCAA
>SEQ ID NO:42 OsACC-A1884 target sequence
   TGGAAGTGCTGCTATTGCCA
>SEQ ID NO:43 OsACC-P1927 target sequence
   ACCAGCCTATTATTCTTACA
>SEQ ID NO:44 OsACC-P2181 target sequence
   AAGATAAACCCAGACCGCAT
>SEQ ID NO:45 wildtype OsHPPD 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRRAGDVLSEEQINECQELGVLVDRDDQGVLLQIFTKPVGDRPTFFLEM
IQRIGCMEKDESGQEYQKGGCGGFGKGNFSELFKSIEEYEKSLEAKQAPTVQGS*
>SEQ ID NO:46 OsHPPD-M1(L365F) 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRRAGDVLSEEQINECQELGVFVDRDDQGVLLQIFTKPVGDRPTFFLEM
IQRIGCMEKDESGQEYQKGGCGGFGKGNFSELFKSIEEYEKSLEAKQAPTVQGS*
>SEQ ID NO:47 OsHPPD-M2(T379I) 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRRAGDVLSEEQINECQELGVLVDRDDQGVLLQIFIKPVGDRPTFFLEMI
QRIGCMEKDESGQEYQKGGCGGFGKGNFSELFKSIEEYEKSLEAKQAPTVQGS*
>SEQ ID NO:48 OsHPPD-M3(E353K/E354K) 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRRAGDVLSKKQINECQELGVLVDRDDQGVLLQIFTKPVGDRPTFFLEM
IQRIGCMEKDESGQEYQKGGCGGFGKGNFSELFKSIEEYEKSLEAKQAPTVQGS*
>SEQ ID NO:49 OsHPPD-M4(R346C/A347V) 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRCVGDVLSEEQINECQELGVLVDRDDQGVLLQIFTKPVGDRPTFFLEM
IQRIGCMEKDESGQEYQKGGCGGFGKGNFSELFKSIEEYEKSLEAKQAPTVQGS*
>SEQ ID NO:50 OsHPPD-M5D369N/D370N 
MPPTPTPTATTGAVSAAAAAGENAGFRLVGHRRFVRANPRSDRFQALAFHHVELWCADAASAAG
RFAFALGAPLAARSDLSTGNSAHASLLLRSASVAFLFTAPYGGDHGVGADAATTASIPSFSPGAARR
FAADHGLAVHAVALRVADAADAFRASVAAGARPAFQPADLGGGFGLAEVELYGDVVLRFVSHPDG
ADAPFLPGFEGVSNPGAVDYGLRRFDHVVGNVPELAPVAAYISGFTGFHEFAEFTAEDVGTAESGL
NSVVLANNAETVLLPLNEPVHGTKRRSQIQTYLDHHGGPGVQHIALASDDVLGTLREMRARSAM
GGFEFLAPPPPNYYDGVRRRAGDVLSEEQINECQELGVLVDRNNQGVLLQIFTKPVGDRPTFFLEM
IQRIGCMEKDESGQEYQKGGCGGFGKGNFSELFKSIEEYEKSLEAKQAPTVQGS*
>SEQ ID NO:51 rice HPPD encoding sequence 
ATGCCTCCCACTCCCACCCCCACCGCCACCACCGGCGCCGTCTCGGCCGCTGCGGCGGCGGGG
GAGAACGCGGGGTTCCGCCTCGTCGGGCACCGCCGCTTCGTCCGCGCCAACCCGCGGAGCGA
CCGGTTCCAGGCGCTCGCGTTCCACCACGTCGAGCTCTGGTGCGCCGACGCCGCGTCCGCCGC
GGGCCGGTTCGCCTTCGCCCTGGGCGCGCCGCTCGCCGCCAGGTCCGACCTCTCCACGGGGAA
CTCCGCGCACGCCTCCCTCCTCCTCCGCTCCGCCTCCGTCGCGTTCCTCTTCACCGCCCCCTAC
GGCGGCGACCACGGCGTCGGCGCGGACGCGGCCACCACCGCCTCCATCCCTTCCTTCTCCCCA
GGCGCCGCGCGGAGGTTCGCCGCGGACCACGGCCTCGCGGTGCACGCCGTGGCGCTGCGCGT
CGCCGACGCGGCCGACGCCTTCCGCGCCAGCGTCGCGGCCGGTGCGCGCCCGGCGTTCCAGCC
CGCCGACCTCGGCGGTGGCTTCGGCCTCGCGGAGGTGGAGCTCTACGGCGACGTCGTGCTCCG
CTTCGTCAGCCACCCGGACGGCGCCGACGCGCCCTTCCTCCCGGGTTTCGAGGGCGTCAGCAA
CCCGGGCGCCGTGGACTACGGCCTCCGCCGGTTCGACCACGTCGTCGGCAACGTGCCGGAGCT
CGCTCCGGTAGCCGCGTACATCTCCGGGTTCACCGGGTTCCACGAGTTCGCCGAGTTCACCGCC
GAGGACGTGGGCACCGCCGAGAGCGGCCTCAACTCGGTGGTGCTCGCCAACAACGCGGAGAC
CGTGCTGCTGCCGCTCAACGAGCCGGTGCACGGCACCAAGCGGCGGAGCCAGATACAGACGT
ACCTGGACCACCACGGCGGCCCGGGGGTGCAGCACATCGCGCTGGCCAGCGACGACGTGCTC
GGGACGCTGAGGGAGATGCGGGCGCGCTCCGCCATGGGCGGCTTCGAGTTCTTGGCGCCGCCG
CCGCCCAACTACTACGACGGCGTGCGGCGGCGCGCCGGGGACGTGCTCTCGGAGGAGCAGAT
CAACGAGTGCCAGGAGCTCGGGGTGCTCGTGGACAGGGATGACCAGGGGGTGTTGCTCCAGA
TCTTCACCAAGCCAGTAGGAGACAGGCCAACCTTTTTCTTGGAGATGATACAAAGGATTGGGT
GCATGGAGAAGGATGAGAGTGGGCAGGAGTACCAGAAGGGCGGCTGCGGCGGGTTTGGGAAG
GGCAACTTCTCGGAGCTGTTCAAGTCCATTGAGGAGTATGAGAAATCCCTTGAAGCCAAGCAA
GCCCCTACAGTTCAAGGATCCTAG
>SEQ ID NO:52 target sequence 1
   GTGCGGCGGCGCGCCGGGGA
>SEQ ID NO:53 target sequence 2
   GCGCGCCGGGGACGTGCTCT
>SEQ ID NO:54 target sequence 3
   GGACGTGCTCTCGGAGGAGC
>SEQ ID NO:55 target sequence 4
   CTCGGAGGAGCAGATCAACG
>SEQ ID NO:56 target sequence 5
   TGCCAGGAGCTCGGGGTGCT
>SEQ ID NO:57 target sequence 6
   AGCTCGGGGTGCTCGTGGAC
>SEQ ID NO:58 target sequence 7
   TGACCAGGGGGTGTTGCTCC
>SEQ ID NO:59 target sequence 8
   GTTGCTCCAGATCTTCACCA
>SEQ ID NO:60 target sequence 9
   TCACCAAGCCAGTAGGAGAC
>SEQ ID NO:61 target sequence 10
   GCCAACCTTTTTCTTGGAGA
>SEQ ID NO:62 target sequence 11
   GATACAAAGGATTGGGTGCA
>SEQ ID NO:63 target sequence 12
   AGTGGGCAGGAGTACCAGAA
>SEQ ID NO:64 target sequence 13
   GAGTACCAGAAGGGCGGCTG
>SEQ ID NO:65 target sequence 14
   CAACTTCTCGGAGCTGTTCA
>SEQ ID NO:66 target sequence15
   CAAGTCCATTGAGGAGTATG
>SEQ ID NO:67 target sequence16
   AAATCCCTTGAAGCCAAGCA
>SEQ ID NO:68 target sequence17
   GAAGCCAAGCAAGCCCCTAC
>SEQ ID NO:69 target sequence18
   CTTGAAGCCAAGCAAGCCCC
>SEQ ID NO:70 target sequence19
   AGTCCATTGAGGAGTATGAG
>SEQ ID NO:71 target sequence20
   ACTTCTCGGAGCTGTTCAAG
>SEQ ID NO:72 target sequence21
   GCGGCGGGTTTGGGAAGGGC
>SEQ ID NO:73 target sequence22
   GGAGTACCAGAAGGGCGGCT
>SEQ ID NO:74 target sequence23
   GAAGGGCGGCTGCGGCGGGT
>SEQ ID NO:75 target sequence24
   TGGAGAAGGATGAGAGTGGG
>SEQ ID NO:76 target sequence25
   AAGGATTGGGTGCATGGAGA
>SEQ ID NO:77 target sequence26
   TGGAGATGATACAAAGGATT
>SEQ ID NO:78 target sequence27
   ACCTTTTTCTTGGAGATGAT
>SEQ ID NO:79 target sequence28
   GCTATTTCACTCAGGCCAAC
>SEQ ID NO:80 target sequence29
   TCACCAAGCCAGTAGGAGAC
>SEQ ID NO:81 target sequence30
   GGGATGACCAGGGGGTGTTG
>SEQ ID NO:82 target sequence31
   CGTGGACAGGGATGACCAGG
>SEQ ID NO:83 target sequence32
   CGGGGTGCTCGTGGACAGGG
>SEQ ID NO:84 target sequence33
   AGTGCCAGGAGCTCGGGGTG
>SEQ ID NO:85 target sequence34
   GATCAACGAGTGCCAGGAGC
>SEQ ID NO:86 target sequence35
   GGGACGTGCTCTCGGAGGAG
>SEQ ID NO:87 target sequence36
   GCGGCGCGCCGGGGACGTGC
>SEQ ID NO:88 target sequence37
   GCGTGCGGCGGCGCGCCGGG
>SEQ ID NO:89 A3A-NG-PBE

MWIFLALPSYAIYLLGTVSFVDAHPVVWCYFCSPRMEASPASGPRHLMDPHIFTSNFNNGIGRHKT
YLCYEVERLDNGTSVKMDQHRGFLHNQAKNLLCGFYGRHAELRFLDLVPSLQLDPAQIYRVTWFI
SWSPCFSWGCAGEVRAFLQENTHVRLRIFAARIYDYDPLYKEALQMLRDAGAQVSIMTYDEFKHC
WDTFVDHQGCPFQPWDGLDEHSQALSGRLRAILQNQGNSGSETPGTSESATPESRPDKKYSIGLAI

GTNSVGWAVITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIYHLRKKLVDS
TDKADLRLIYLALAHMIKFRGHFLIEGDLNPDNSDVDKLFIQLVQTYNQLFEENPINASGVDAKAIL
SARLSKSRRLENLIAQLPGEKKNGLFGNLIALSLGLTPNFKSNFDLAEDAKLQLSKDTYDDDLDNL
LAQIGDQYADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSEETITPWNFEE
VVDKGASAQSFIERMTNFDKNLPNEKVLPKHSLLYEYFTVYNELTKVKYVTEGMRKPAFLSGEQK
KAIVDLLFKTNRKVTVKQLKEDYFKKIECFDSVEISGVEDRFNASLGTYHDLLKIIKDKDFLDNEEN
EDILEDIVLTLTLFEDREMIEERLKTYAHLFDDKVMKQLKRRRYTGWGRLSRKLINGIRDKQSGKTI
LDFLKSDGFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVENTQLQNEKL
YLYYLQNGRDMYVDQELDINRLSDYDVDHIVPQSFLKDDSIDNKVLTRSDKNRGKSDNVPSEEVV
KKMKNYWRQLLNAKLITQRKFDNLTKAERGGLSELDKAGFIKRQLVETRQITKHVAQILDSRMNT
KYDENDKLIREVKVITLKSKLVSDFRKDFQFYKVREINNYHHAHDAYLNAVVGTALIKKYPKLESE
FVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESIRPKRNSDKLIARKKDWDPKKYGGFVSPTV
AYSVLVVAKVEKGKSKKLKSVKELLGITIMERSSFEKNPIDFLEAKGYKEVKKDLIIKLPKYSLFELE
NGRKRMLASARFLQKGNELALPSKYVNFLYLASHYEKLKGSPEDNEQKQLFVEQHKHYLDEIIEQI
SEFSKRVILADANLDKVLSAYNKHRDKPIREQAENIIHLFTLTNLGAPRAFKYFDTTIDRKVYRSTK
EVLDATLIHQSITGLYETRIDLSQLGGDKRPAATKKAGQAKKKKTRDSGGSTNLSDIIEKETGKQLVI
QESILMLPEEVEEVIGNKPESDILVHTAYDESTDENVMLLTSDAPEYKPWALVIQDSNGENKIKMLS
GGSPKKKRKV

## Claims

1. An ALS mutant or a functional fragment thereof having an amino acid mutation at one or more positions selected from the group consisting of positions 99, 98, 101 and 102 relative to wild-type ALS, with the amino acid positions referring to SEQ ID NO : 1.

2. The ALS mutant or functional fragment thereof of claim 1, which, when expressed in a plant, is capable of conferring herbicide resistance to the plant.

3. The ALS mutant or a functional fragment thereof of claim 2, wherein the herbicide is selected from the group consisting of sulfonylureas (SU), imidazolidinones (IMI), triazolopyrimidines (TP), pyrimidinylthiobenzoates (PTB) and sulfonamido-carbonyl-triazolinone (SCT) compounds, or any combination thereof.

4. The ALS mutant or functional fragment thereof of claim 1, wherein relative to wild-type ALS, the ALS mutant or functional fragment thereof comprises one or more amino acid substitutions selected from the group consisting of A99T, G98N, M101I, E102K, with the amino acid positions referring to SEQ ID NO:1.

5. The ALS mutant or functional fragment thereof of claim 1, comprising the amino acid sequence shown in any one of SEQ ID NOs: 4-8.

6. A PPO mutant or a functional fragment thereof having an amino acid mutation at one or more positions selected from the group consisting of positions 127 and 129 relative to wild-type PPO, with the amino acid positions referring to SEQ ID NO:9.

7. The PPO mutant or functional fragment thereof of claim 6, which, when expressed in a plant, is capable of conferring herbicide resistance to the plant.

8. The PPO mutant or functional fragment thereof of claim 7, wherein the herbicide is selected from diphenyl ethers, cyclic imines, phenylpyrazoles, bicyclic triazolones, or any of them combination.

9. The PPO mutant or functional fragment thereof of claim 6, wherein relative to wild-type PPO, the PPO mutant or functional fragment thereof comprises one or more amino acid substitutions selected from from the group consisting of R127C, R127G, T129I, with the amino acid positions referring to SEQ ID NO:9.

10. The PPO mutant or functional fragment thereof of claim 6, comprising the amino acid sequence shown in any one of SEQ ID NOs: 12-16.

11. An ACCase mutant or a functional fragment thereof, having an amino acid mutation at one or more positions selected from the group consisting of positions 1866, 1884, 1927 and 2181 relative to wild-type ACCase, with the amino acid positions referring to SEQ ID NO:31.

12. The ACCase mutant or functional fragment thereof according to claim 11, which, when expressed in a plant, is capable of conferring herbicide resistance to the plant.

13. The ACCase mutant or functional fragment thereof of claim 12, wherein the herbicide is selected from aryloxyphenoxypropionates, cyclohexanediones or phenylpyrazolines, or any combination thereof.

14. The ACCase mutant or functional fragment thereof of claim 11, wherein relative to wild-type ACCase, the ACCase mutant or functional fragment thereof comprises one or more amino acid substitutions selected from the group consisting of S1866F, A1884P, P1927, P2181L, with the amino acid positions referring to SEQ ID NO:31.

15. The ACCase mutant or functional fragment thereof of claim 11, which comprises the amino acid sequence shown in any one of SEQ ID NOs: 32-35 or 37-40.

16. A HPPD mutant or a functional fragment thereof having an amino acid mutation at one or more positions selected from the group consisting of positions 365, 379, 353, 354, 346, 347, 369 or 370 relative to wild-type HPPD, with the amino acid positions referring to SEQ ID NO:45.

17. The HPPD mutant or functional fragment thereof of claim 16, which, when expressed in a plant, is capable of conferring herbicide resistance to the plant.

18. The HPPD mutant or functional fragment thereof of claim 17, wherein the herbicide is selected from the group consisting of pyrazoles, triketones, isoxazoles, diketonitriles and benzophenones, or any combination thereof.

19. The HPPD mutant or functional fragment thereof of claim 16, wherein relative to wild-type HPPD, the HPPD mutant or functional fragment thereof comprises one or more amino acid substitutions selected from L365F, T379I, E353K, E354K, E353K/E354K, R346C, A347V, R346C/A347V, D369N, D370N, D369N/D370N, with the amino acid positions referring to SEQ ID NO:45.

20. The HPPD mutant or functional fragment thereof of claim 16, said HPPD mutant comprises the amino acid sequence shown in any one of SEQ ID NOs: 46-50.

21. A nucleic acid, which comprises a nucleotide sequence encoding the ALS mutant or a functional fragment thereof of any one of claims 1-5 and/or a nucleotide sequence encoding the PPO mutant or a functional fragment thereof of any one of claims 6-10 and/or a nucleotide sequence encoding the ACCase mutant or a functional fragment thereof of any one of claims 11-15 and/or a nucleotide sequence encoding the HPPD mutant or a functional fragment thereof any of the claims 16-20.

22. An expression cassette, which comprises a nucleotide sequence encoding the ALS mutant or a functional fragment thereof of any one of claims 1-5 and/or a nucleotide sequence encoding the PPO mutant or a functional fragment thereof of any one of claims 6-10 and/or a nucleotide sequence encoding the ACCase mutant or a functional fragment thereof of any one of claims 11-15 and/or a nucleotide sequence encoding the HPPD mutant or a functional fragment thereof any of the claims 16-20, operably linked to a regulatory sequence.

23. An expression construct comprising the expression cassette of claim 22.

24. A method for production of a herbicide-resistant plant through transgene, comprising introducing the nucleic acid of claim 21, the expression cassette of claim 22 and/or the expression construct of claim 23 into the plant.

25. A method for generating a herbicide-resistant plant by base editing, comprising introducing into the plant a base editing system targeting an endogenous ALS coding region and/or an endogenous PPO coding region and/or an endogenous ACCase coding region and/or an endogenous HPPD coding region in the genome of the plant, thereby resulting in
(i) amino acid mutation in the endogenous ALS at one or more positions selected from the group consisting of positions 99, 98, 101 and 102, said ALS amino acid position referring to SEQ ID NO: 1, and/or
(ii) amino acid mutation in the endogenous PPO at one or more positions selected from the group consisting of positions 127 and 129, the PPO amino acid positions referring to SEQ ID NO: 9, and/or
(iii) amino acid mutation in the endogenous ACCase at one or more positions selected from the group consisting of positions 1866, 1884, 1927 or 2181, said ACCase amino acid positionsreferring to SEQ ID NO: 31, and/or
(iv) amino acid mutation in the endogenous HPPD at one or more positions selected from the group consisting of positions 365, 379, 353, 354, 346, 347, 369 or 370, the HPPD amino acid position referring to SEQ ID NO:45.

26. The method of claim 25, wherein introduction of the base editing system results in the endogenous ALS comprising one or more amino acid substitutions selected from the group consisting of A99T, G98N, M101I, E102K, the ALS amino acid positions referring to SEQ ID NO:1

27. The method of claim 25, wherein introduction of the base editing system results in the endogenous PPO comprising one or more amino acid substitutions selected from the group consisting of R127C, R127G, T129I, the PPO amino acid positions referring to SEQ ID NO:9.

28. The method of claim 25, wherein introduction of the base editing system results in the endogenous ACCase comprising one or more amino acid substitutions selected from the group consisting of S1866F, A1884P, P1927, P2181L, the ACCase amino acid positions referring to SEQ ID NO:31.

29. The method of claim 25, wherein introduction of the base editing system results in the endogenous HPPD comprising one or more amino acid substitutions selected from the group consisting of L365F, T379I, E353K, E354K, E353K/E354K, R346C, A347V, R346C/A347V, D369N, D370N, D369N/D370N, the HPPD amino acid positions referring to SEQ ID NO:45.

30. The method of any one of claims 25-29, wherein the base editing system comprises a base editing fusion protein or an expression construct comprising a nucleotide sequence encoding the base editing fusion protein, and at least one guide RNA or an expression construct comprising a nucleotide sequence encoding at least one guide RNA, wherein the base editing fusion protein comprises a CRISPR effector protein and a deaminase domain, and the at least one guide RNA can target the base editing fusion protein to an endogenous ALS coding sequence and/or an endogenous PPO coding sequence and/or an endogenous ACCase coding sequence and/or an endogenous HPPD coding sequence in the plant genome.

31. The method of claim 30, the base editing fusion protein comprises the amino acid sequence set forth in SEQ ID NO: 24 or 25, the at least one guide RNA comprises a nucleotide sequence selected from the group consisting of SEQ ID NOs: 26-30, 41-44 and 52-88, or the complement thereof.

32. The method of any one of claims 24-31, wherein the plant is a monocotyledonous or dicotyledonous plant, preferably the plant is a crop plant, such as a monocotyledonous crop plant.

33. The method of claim 32, wherein the plant is selected from the group consisting of rice, wheat, barley, sorghum, corn, preferably the plant is wheat or rice.
